(19) Europäisches Patentamt European Patent Office Office européen des brevets

(11) **EP 4 555 927 A1**

(12) **EUROPEAN PATENT APPLICATION**

(43) Date of publication:
**21.05.2025 Bulletin 2025/21**

(21) Application number: **24208150.3**

(22) Date of filing: **22.10.2024**

(51) International Patent Classification (IPC):
**A61B 5/145** (2006.01)   **A61B 5/1495** (2006.01)

(52) Cooperative Patent Classification (CPC):
**A61B 5/14532; A61B 5/1495;** A61B 2562/12

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.10.2023 PCT/GR2023/000058**
**08.10.2024 US 202418908892**

(71) Applicant: **Medtronic MiniMed, Inc.**
**Northridge, CA 91325 (US)**

(72) Inventors:
• **Nava-Gurerra, Leonardo**
**Northridge, 91325 (US)**
• **Emig, Molly E.**
**Northridge, 91325 (US)**
• **Arguelles Morales, Juan Enrique**
**Northridge, 91325 (US)**
• **Kanale, Anup V.**
**Northridge, 91325 (US)**

• **Qiu, Kelly Joy**
**Northridge, 91325 (US)**
• **Sharifi Sarabi, Mona M**
**Northridge, 91325 (US)**
• **Tran, Chi A**
**Northridge, 91325 (US)**
• **Haas, Anthony**
**Northridge, 91325 (US)**
• **Piccinini, Francesca**
**Northridge, 91325 (US)**
• **Notghi, Bahram B.**
**Northridge, 91325 (US)**
• **Mallas, Georgios**
**Northridge, 91325 (US)**
• **Zhang, Yi**
**Northridge, 91325 (US)**
• **Shah, Zachary Andrew**
**Northridge, CA (US)**

(74) Representative: **Zimmermann & Partner**
**Patentanwälte mbB**
**Postfach 330 920**
**80069 München (DE)**

(54) **INTEGRATION OF IN VIVO PREDICTIVE MODEL OUTPUT FEATURES FOR CGM ALGORITHM PERFORMANCE IMPROVEMENT**

(57) Techniques disclosed herein relate to glucose level measurement and/or management. In some embodiments, the techniques involve obtaining in vivo characteristics of a glucose sensor predicted using fabrication process measurement data associated with the glucose sensor, the in vivo characteristics including an in vivo sensitivity, an in vivo intercept, or a combination thereof; receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and estimating a sensor glucose (SG) value using an SG model, wherein input parameters of the SG model include the in vivo characteristics of the glucose sensor and the sensor measurement data, and the SG value is an output of the SG model.

*FIG. 9*

**Description**

TECHNICAL FIELD

The present disclosure relates generally to glucose level measurement and/or management.

BACKGROUND

The pancreas of a healthy person can produce and release insulin into the blood stream in response to elevated blood glucose levels. More specifically, beta cells ($\beta$-cells) in the pancreas can produce and secrete insulin into the blood stream as needed. If $\beta$-cells become incapacitated or die, a condition known as Type 1 diabetes mellitus, insulin may need to be provided to the diabetic patient's body using, for example, a syringe, a pen, or an infusion pump of a blood glucose level management system, to maintain health or life.

Some blood glucose level management systems may be closed-loop systems that may include a pump automatically or semi-automatically controlled to deliver insulin to the patient. Some blood glucose level management systems may require manual administration of insulin based on dosage determined by an insulin calculator. The delivery of insulin to the patient can be controlled to occur at time instants and in amounts that are determined based on, for example, the amount of carbohydrates taken by a patient and/or real-time measurements of glucose levels by a glucose sensor. Glucose sensors may detect and/or quantify the amount of glucose in a patient's body (e.g., interstitial glucose or blood glucose), which enables patients and medical personnel to monitor physiological conditions within the patient's body. In many circumstances (e.g., in a diabetic patient), it may be beneficial to monitor glucose levels on a continuing basis. Such information can be useful in monitoring and/or adjusting a treatment regimen, which typically includes administration of insulin to the patient. Rather than continuously sampling and monitoring a user's blood glucose level, which may compromise battery life, intermittently sensed glucose data samples may often be utilized for purposes of continuous glucose monitoring (CGM) or determining operating commands for the infusion pump.

SUMMARY

This disclosure relates generally to glucose level measurement and/or management. More specifically, techniques disclosed herein relate to estimating blood glucose levels using measurement data from a subcutaneous sensor and in vivo characteristics of the subcutaneous sensor predicted based on fabrication process measurement data. The techniques disclosed herein may be practiced in a variety of ways, such as using a processor-implemented method, a system comprising one or more processors and one or more processor-readable media, and/or one or more (non-transitory) processor-readable media.

According to certain embodiments, the techniques involve obtaining in vivo characteristics of a glucose sensor predicted using fabrication process measurement data associated with the glucose sensor, the in vivo characteristics including an in vivo sensitivity, an in vivo intercept, or a combination thereof; receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and estimating a sensor glucose (SG) value using an SG model, wherein input parameters of the SG model include the in vivo characteristics of the glucose sensor and the sensor measurement data, and the SG value is an output of the SG model.

This summary is neither intended to identify key or essential features of the claimed subject matter, nor is it intended to be used in isolation to determine the scope of the claimed subject matter. The subject matter should be understood by reference to appropriate portions of the entire specification of this disclosure, any or all drawings, and each claim. The foregoing, together with other features and examples, will be described in more detail below in the following specification, claims, and accompanying drawings.

In an embodiment, techniques disclosed herein relate to glucose level measurement and/or management. In some embodiments, the techniques involve obtaining in vivo characteristics of a glucose sensor predicted using fabrication process measurement data associated with the glucose sensor, the in vivo characteristics including an in vivo sensitivity, an in vivo intercept, or a combination thereof; receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and estimating a sensor glucose (SG) value using an SG model, wherein input parameters of the SG model include the in vivo characteristics of the glucose sensor and the sensor measurement data, and the SG value is an output of the SG model.

BRIEF DESCRIPTION OF THE DRAWINGS

Illustrative embodiments are described in detail below with reference to the following figures.

FIG. 1 illustrates an example of a blood glucose level management system according to certain embodiments.

FIG. 2 is a block diagram of an example of a glucose control system according to certain embodiments.

FIG. 3 illustrates an example of a continuous glucose monitoring (CGM) system according to certain embodiments.

FIG. 4A illustrates an example of a glucose sensor according to certain embodiments.

FIG. 4B illustrates another example of a glucose sensor according to certain embodiments.

FIG. 4C illustrates a cross-section of an example of a working electrode of a glucose sensor according to certain embodiments.

FIG. 5 shows a schematic of a glucose sensor according to certain embodiments.

FIG. 6 includes a flow diagram illustrating an example of a method of determining sensor glucose values using measurement data from a glucose sensor, in accordance with certain embodiments.

FIG. 7 illustrates an example of a sensor feature generator according to certain embodiments.

FIG. 8 illustrates an example of determining a sensor glucose value using one or more models according to certain embodiments.

FIG. 9 illustrates an example of determining a translation model for mapping in vitro characteristics of a CGM sensor to in vivo characteristics of the CGM sensor according to certain embodiments.

FIG. 10 illustrates an example of determining in vitro and/or in vivo characteristics of glucose sensors using fabrication process measurement data according to certain embodiments.

FIG. 11A shows an example of a partitioned model including a set of regional models for modeling a relationship between sensor glucose (SG) values and sensor measurement data according to certain embodiments.

FIG. 11B shows another example of a partitioned model including a set of regional models for modeling a relationship between SG values and sensor measurement data according to certain embodiments.

FIG. 11C illustrates an example of an ensembled model including multiple sets of regional models for estimating SG values based on sensor measurement data generated by a glucose sensor according to certain embodiments.

FIG. 12A includes a flowchart illustrating an example of a process of predicting in vivo characteristics of a glucose sensor according to certain embodiments.

FIG. 12B includes a flowchart illustrating an example of a process of estimating SG values using in vivo characteristics of a glucose sensor and one or more SG models according to certain embodiments.

FIG. 13A includes a diagram illustrating examples of improving sensor performance by using predicted in vivo characteristics in SG value estimation according to certain embodiments.

FIG. 13B includes a diagram illustrating examples of improving sensor performance by using predicted in vivo characteristics in SG value estimation according to certain embodiments.

FIGS. 14A and 14B depict an example of an integrated glucose sensor device according to certain embodiments, which may implement some of the examples disclosed herein.

FIG. 15 depicts an example of a delivery device, which may implement some of the examples disclosed herein.

FIG. 16 is a simplified block diagram of an example of an electronic device that may implement some of the examples disclosed herein.

The figures depict embodiments of the present disclosure for purposes of illustration only. One skilled in the art will readily recognize from the following description that alternative embodiments of the structures and methods illustrated may be employed without departing from the principles, or benefits touted, of this disclosure.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

DETAILED DESCRIPTION

This disclosure relates generally to glucose level measurement and/or management. More specifically, techniques disclosed herein relate to estimating blood glucose levels using measurement data from a subcutaneous sensor and in vivo characteristics of the subcutaneous sensor predicted based on fabrication process measurement data. The techniques disclosed herein may be practiced in a variety of ways, such as using a processor-implemented method, a system comprising one or more processors and one or more processor-readable media, and/or one or more (non-transitory) processor-readable media.

Diabetes mellitus is a disease of the glucose regulatory system of a patient, where the naturally produced insulin in the body may not be sufficient to control the glucose level in the blood stream of the patient, due to insufficient production of insulin and/or insulin resistance. Therefore, a diabetic patient may need to receive insulin from a pump or another delivery device, such as an injection or infusion device (e.g., a syringe), to control the glucose level in the patient's blood stream. To control the glucose level, a diabetic patient's therapy routine may generally include dosages of basal insulin and bolus insulin. Basal insulin, also referred to as background insulin, may include continuous or constant release of small amounts of insulin to keep blood glucose levels at consistent levels during long time periods. Bolus insulin may be taken specifically before, at, or after mealtimes or other times where there may be a rapid increase in the blood glucose level.

The dose of insulin to be delivered may be determined based on, for example, the carbohydrate count of a meal, and/or the glucose levels of the patient measured using a glucose monitor, such as a fingerstick blood glucose measurement device or a continuous glucose monitoring (CGM) sensor. A CGM sensor may use measurement results (e.g., currents, voltage levels, resistance, etc.) from a sensor inserted into the subcutaneous layer of a user to estimate the blood glucose level of the user based on the glucose level in the interstitial fluid (ISF). In some implementations, a glucose sensor may produce signals through a series of electrochemical reactions when the sensor is inserted and exposed to glucose and oxygen diffused from the interstitial fluid. For example, a glucose sensor may include a glucose limiting membrane (GLM) that limits the amount of glucose and oxygen delivered to a glucose oxidase (GOx) layer of a working electrode of the glucose sensor to ensure that the reactions are glucose limited. The GOx layer or another active enzyme layer on the working electrode of the glucose sensor may break down glucose and oxygen into gluconic acid and hydrogen peroxide. The generated hydrogen peroxide molecules may interact with the working electrode (e.g., with a voltage level applied) to break down hydrogen peroxide into two hydrogen ions, oxygen, and two electrons at the surface of the working electrode. The electrical charges may be forced to move between electrodes due to the potential difference, thereby generating a sensor current signal (Isig) that can be measured by sensor electronics. Other signals such as counter voltage (Vcntr, the voltage potential difference between the counter electrode and the working electrode), electrochemical impedance spectroscopy (EIS) at different frequencies, and the like, may also be measured. The signals measured using the sensor, including Isig, Vcntr, and EIS, may be processed (e.g., filtered, calibrated, or otherwise transformed) to generate some other signals or parameters, such as filtered Isig signals, real and imaginary impedances at various frequencies, derivative signals, and the like. The raw signals measured using the sensor and/or the processed signals may be used in one or more sensor glucose (SG) models to determine SG values that may be estimations of the blood glucose (BG) levels of the user.

The one or more SG models may include one or more trained machine learning models, equations, functions, and the like that may map input parameters (e.g., raw and/or processed sensor measurement data) to SG (or BG) values. For example, an SG model may be represented by:

$$SG = f(Age, Isig, Vcntr, EIS),$$

where age may be the time of the glucose sensor in operation after the insertion. In a simplified example, SG values may be calculated according to:

$$SG = \alpha \times Age + \beta \times Isig + \gamma \times Vcntr + \delta \times EIS + \Delta,$$

where $\alpha$, $\beta$, $\gamma$, and $\delta$ may be coefficients associated with the age, Isig, Vcntr, and EIS, and $\Delta$ is an offset value. In some embodiments, at least some of the coefficients may include vectors or tensors, and at least some of the age, Isig, Vcntr, and EIS may include vectors or tensors.

Sensor properties associated with a glucose sensor, such as the age (or time of use or wear time), battery level, calibration accuracy, sensing environment (e.g., oxygen level, temperature, moisture, and pH value, etc.), physiological dynamics, manufacturing variability, and other properties, may affect the enzymatic glucose level measurement results in complex ways that may be difficult to model. Therefore, many CGM sensors may have suboptimal accuracy and may exhibit large variance in the measurement results. The lower than desired performance of the CGM sensors may be at least partially attributed to sensor-to-sensor manufacturing variability, degradation of the sensor performance over time, complexity of modeling the in vivo behavior of the CGM sensors (e.g., it may be difficult to model the CGM sensor using a same model), and the like.

To achieve the desired accuracy and reliability and reduce the impact of noise and other spurious signals, CGM sensors may need to be periodically or occasionally calibrated in vitro and/or in vivo to improve the accuracy and/or reliability of the glucose sensors. For example, the sensor data may need to be calibrated using a known good blood glucose value, often obtained via a so-called "fingerstick measurement" using a blood glucose meter that measures the blood glucose level in the capillaries. Performing such calibration measurements may increase the patient's burden and perceived complexity, and can be inconvenient, uncomfortable, or otherwise disfavored by patients. Moreover, ISF glucose measurements may lag behind the blood glucose measurements due to the time it takes glucose to diffuse from the capillary to the interstitial space, which may require certain signal processing (e.g., filtering) or other techniques to compensate for the physiological lag. Additionally, various factors can lead to transient changes in the sensor output, which may influence the accuracy of the calibration and the measurement. Degradation of sensor performance over time, which may also vary across sensors due to, for example, manufacture variations between the sensors, may further compound these challenges.

According to certain embodiments disclosed herein, some in vivo characteristics (e.g., in vivo sensor sensitivity and intercept) of a glucose sensor may be predicted before sensor insertion and may be used in one or more SG models to estimate SG values based additionally on other input data, such as Isig, Vcntr, EIS, age, and features derived therefrom Isig, Vcntr, EIS, and/or age. For example, the in vivo characteristics of a glucose sensor may be predicted using in vitro characteristics of the glucose sensor and an in vitro to in vivo translation model, where the in vitro characteristics of the glucose sensor may be predicted using fabrication process measurement data associated with the glucose sensor or may be measured in vitro. In some embodiments, the in vivo sensor characteristics may be predicted directly using the fabrication process measurement data associated with the glucose sensor. The in vivo characteristics may be predicted as a function of time (e.g., the age of the glucose sensor). In this way, at least some of the manufacturing variability and degradation of the glucose sensor can be accounted for in the SG calculation, and thus the SG values may be more accurately determined from raw sensor measurement data (e.g., Isig, Vcntr, EIS, and age).

In one example, fabrication process measurement data associated with an electrochemical glucose sensor, such as, for example, process parameters, thicknesses of various layers of the sensors, material compositions and/or concentrations, certain impedance and/or capacitance measurements, and the like, may be obtained during and/or after the fabrication, and may be used to determine in vitro characteristics of the electrochemical glucose sensor, such as in vitro sensitivity and intercept that may be used to estimate in vitro SG values based on Isig. In vivo characteristics (e.g., in vivo sensitivity and intercept) of the electrochemical glucose sensor may then be determined using the in vitro characteristics of the electrochemical glucose sensor determined using the fabrication process measurement data and one or more translation models. Both the in vitro characteristics and the in vivo characteristics of the electrochemical glucose sensor may vary with the age of the electrochemical glucose sensor and thus may be time-variant (function of time).

Based on the in vivo characteristics (e.g., in vivo sensitivity and in vivo intercept) of the electrochemical glucose sensor, SG values may be determined using one or more SG models that use the in vivo characteristics as part of the inputs in addition to the sensor measurement data. The one or more SG models may include, for example, one or more machine learning models, equations, and the like. For example, one SG model may be represented by:

$$SG = f(Age, Isig, Vcntr, EIS, Sensitivity_{in\_vivo}, Intercept_{in\_vivo}).$$

In a simplified example, SG values may be determined according to:

$$SG = \alpha \times Age + \beta \times \frac{Isig}{Sensitvity_{in\_vivo}} + \gamma \times Vcntr + \delta \times EIS + \theta \times Intercept_{in\_vivo},$$

where $\alpha$, $\beta$, $\gamma$, $\delta$, and $\theta$ are coefficients that may or may not be time-variant and may include individual values, vectors, or matrixes. In some embodiments, an offset value may also be used in the above equation. As a result, the effect of fabrication process variability and sensor degradation over time on the sensor measurement data may be at least partially accounted for by using the time-variant in vivo characteristics of the sensor in the SG models. Therefore, the determined SG values may be more accurate estimations of the blood glucose levels.

In many cases, a single SG model may not be able to accurately estimate the blood glucose level because various sensor properties may affect the glucose level measurement results in complex ways that may be difficult to characterize using a single SG model. According to certain embodiments, different SG models may be used for different input parameter ranges, such as different ranges of the age, Vcntr, or other sensor measurement data. Each SG model may be optimized for a respective region of the input parameter space, and thus may be able to more accurately characterize the response of the sensor to glucose within the respective region.

Aspects and embodiments of the present disclosure can be practiced with one or more types of insulin (e.g., fast-acting insulin, intermediate-acting insulin, and/or slow-acting insulin). Unless indicated otherwise by the context, terms such as "dose," "insulin," "basal," and "bolus" may not denote a particular type of insulin. For example, fast-acting insulin may be used for both basal dosages and bolus dosages. The term "basal" can refer to and include insulin that is delivered in an amount and at a frequency that is intended to correspond to a healthy body's release of insulin between meals and during sleep. The term "bolus" may refer to and include insulin that is delivered in an amount and at a timing that is intended to correspond to a healthy body's release of insulin for counteracting a high glucose level, such as that caused by the consumption of a meal. A meal may include any type or amount of food or beverage consumption, including breakfast, lunch, dinner, snacks, and beverages, among others.

In the following description, for the purposes of explanation, specific details are set forth in order to provide a thorough understanding of examples of the disclosure. However, it will be apparent that various examples may be practiced without these specific details. For example, devices, systems, structures, assemblies, methods, and other components may be shown as components in block diagram form in order not to obscure the examples in unnecessary detail. In other instances, well-known devices, processes, systems, structures, operations, and techniques may be shown without necessary detail or may not be shown, in order to avoid obscuring the examples. The figures and description are not intended to be restrictive. The terms and expressions that have been employed in this disclosure are used as terms of description and not of limitation, and there is no intention in the use of such terms and expressions of excluding any equivalents of the features shown and described or portions thereof. The word "example" is used herein to mean "serving as an example, instance, or illustration." Any embodiment or design described herein as "example" is not necessarily to be construed as preferred or advantageous over other embodiments or designs.

Carbohydrates in food intake may be broken down into glucose (sugar) in the stomach and/or intestine, and may be absorbed at the small intestine and/or large intestine into the blood stream. The blood stream may transport glucose to the capillaries of the body, where some glucose may diffuse into the interstitial fluid between cells (e.g., fat or muscle cells), which may use the glucose for energy. The endocrine system of the human body that directs and regulates the functions and activities of the body (working with the nervous system) may secrete chemicals that transmit messages to tissues and organs. For example, endocrine glands or organs may release hormones into the blood stream for transportation to target cells with receptors. In one example, insulin may be made by the β-cells of pancreas, and may, for example, increase the glucose uptake, enhance glucose utilization, stop hepatic glucose production, stimulate glycogen formation in liver and skeletal muscle, promote protein synthesis, and increase fat storage.

Insulin may function as a key that can unlock cells and help glucose to move into cells where the glucose may be used for energy. Without insulin, glucose may not be able to enter cells to be used for energy, and may build up in the interstitial fluid and blood stream. A healthy pancreas may continuously release a small amount of regular human insulin 24 hours a day, including between meals and during sleep. The small amount pf insulin may match the liver's release of glucose. A healthy pancreas may also secrete a larger amount of insulin after food intake to match the amount of food intake.

The liver of a person may absorb excessive glucose from digestion and convert excessive glucose to glycogen for storage so that glucose may be available when needed. For example, when the blood glucose level is low, the α-cells of the pancreas may secrete glucagon. Glucagon may cause the liver to release the stored form of glucose (glycogen) into the blood stream to help increase the glucose level. The balance between insulin secreted by the β-cells and the glucagon secreted by the α-cells may help to maintain the normal blood glucose level, such as in the range of about 80-120 mg/dL before meals.

The naturally produced insulin in the body of a diabetic patient may not be sufficient to control the glucose level in the blood stream of the patient, due to insufficient production of insulin and/or insulin resistance. Therefore, a diabetic patient may need to receive insulin from a pump or another delivery device such as an injection or infusion device to control the glucose level in the patient's blood stream. To control the glucose level, a diabetic patient's therapy routine may generally include dosages of basal insulin and bolus insulin. Dosages of insulin to be delivered may be determined based on, for example, the carbohydrate count of a meal, and/or the glucose level of the patient measured using a glucose monitor, such as a continuous glucose monitor (CGM).

In some implementations (e.g., in a closed-loop system), the insulin delivery device may communicate with or otherwise use a sensor device (including but not limited to a CGM) to perform various measurements for a patient. In one example, the sensor device may include subcutaneous implanted electrodes to concurrently monitor the patient's response to meals and insulin introduced by the insulin delivery device. The sensor device and the insulin delivery device may be in a communication network (wired or wireless) with one or more processors and/or patient devices (such as a patient's smartphone equipped with an application or other software) to create an overall system for monitoring a patient disease state and for facilitating treatment thereof.

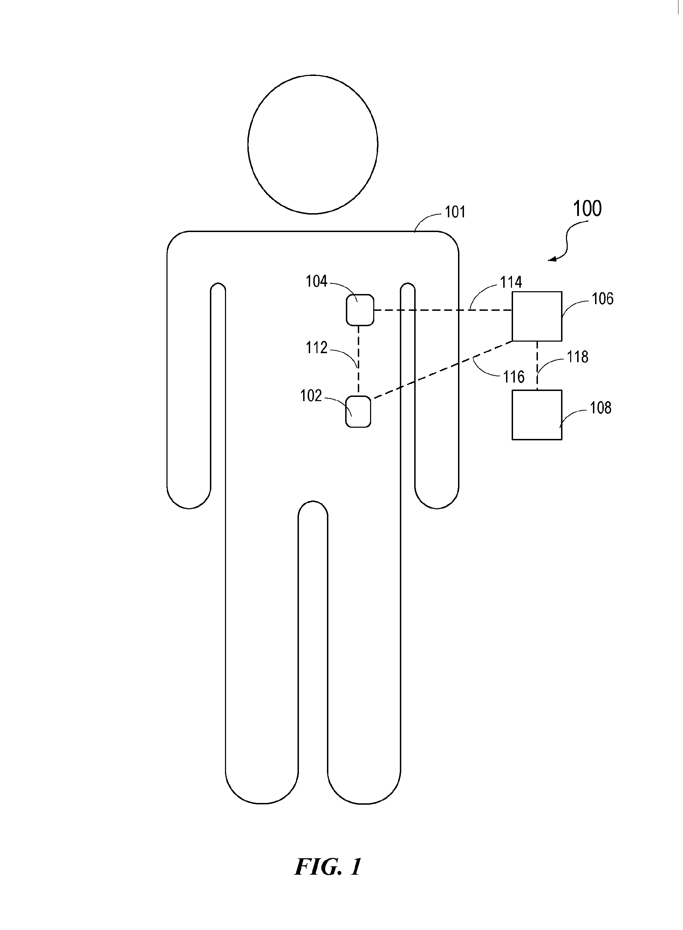

FIG. 1 illustrates an example of a blood glucose level management system 100 according to certain embodiments. Blood glucose level management system 100 may be used to monitor and regulate the blood glucose level of a patient 101. In the illustrated example, blood glucose level management system 100 may include a delivery device 102, a monitoring device 104, a computing device 106, and an optional remote/cloud computing system 108. Delivery device 102, monitoring device 104, and computing device 106 may be embodied in various ways, including being disposed in one or more device housings. For example, in some embodiments, all of devices 102-106 may be disposed in a single device housing. In some embodiments, each of devices 102-106 may be disposed in a separate device housing. In some embodiments, two or more of devices 102-106 may be disposed in the same device housing. In some embodiments, a single device 102, 104, or 106 may have two or more parts that are disposed in two or more housings. For example, monitoring device 104 may include an on-body part and a display and control part communicated with the on-body part through wires or wirelessly. Delivery device 102 may include an on-body site (e.g., including a cannula) and a part that includes a reservoir, a pump, and a control unit. These and other embodiments, and combinations thereof, are contemplated to be within the scope of the present disclosure.

Blood glucose level management system 100 may include a plurality of communication links, such as communication links 112-118. Communications links 112-118 may each be a wired connection and/or a wireless connection. In embodiments where two devices are located in a same housing, the communication link may include, for example, wires, cables, and/or communication buses on a printed circuit board, among other things. In embodiments where two devices are separate from each other in different device housings, the communication links may be wired and/or wireless connections. Wired connections may include, for example, an Ethernet connection, a Universal Serial Bus (USB) connection, and/or another type of physical connection. Wireless connections may include, for example, a cellular connection, a Wi-Fi connection, a Bluetooth® connection, a mesh network connection, and/or another type of connection using a wireless communication protocol. Some embodiments of communication links 112-118 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for communication links 112-118.

Delivery device 102 may be configured to deliver a therapeutic substance to patient 101. The therapeutic substance may include, for example, insulin, HIV drugs, drugs to treat pulmonary hypertension, iron chelation drugs, pain medications, anti-cancer treatments, medications, vitamins, hormones, a nutritional supplement, a dye, a tracing medium, a saline medium, a hydration medium, and the like. Delivery device 102 may be secured to patient 101 (e.g., to the body or clothing of patient 101) or may be at least partially implanted in the body of patient 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of patient 101. The cannula and/or a needle may facilitate delivery of the therapeutic substance to a tissue layer, vein, interstitial fluid, or body cavity of patient 101. During operation, the actuator, in response to a signal (e.g., a command signal), may drive the delivery mechanism, thereby causing the therapeutic substance to move from the reservoir, through the cannula, and into the body of patient 101.

The components of delivery device 102 described above are merely provided as an example. Delivery device 102 may include other components, such as, without limitation, a power supply, a communication transceiver, one or more processors or other computing resources, memory devices, and/or user interfaces (e.g., buttons, keys, display, etc.), among other things. In some implementations, delivery device 102 may host an App (e.g., an insulin calculator) that may calculate the desired amount of therapeutic substance to be delivered to patient 101. Persons skilled in the art will recognize various implementations of delivery device 102 and the components of such implementations. All such implementations and components are contemplated to be within the scope of the present disclosure.

Monitoring device 104 may be configured to detect a physiological condition (e.g., a glucose concentration level) of patient 101 and may also be configured to detect other things. Monitoring device 104 may be secured to the body of patient 101 (e.g., to the skin of patient 101 via an adhesive) and/or may be at least partially implanted into the body of patient 101. Depending on the particular location or configuration, monitoring device 104 may be in contact with biological matter (e.g., interstitial fluid and/or blood) of patient 101.

Monitoring device 104 may include one or more sensors (not shown), such as, without limitation, electrochemical sensors, electrical sensors, and/or optical sensors. As persons skilled in the art will understand, an electrochemical sensor may be configured to respond to the interaction or binding of a biological marker to electrodes by generating an electrical signal based on, for example, a potential, conductance, current, and/or impedance of an electrical path through the electrodes. The electrodes may include a material selected to interact with a particular biomarker, such as glucose. The potential, current, conductance, and/or impedance may correlate with a concentration of the particular biomarker. In one example, the electrochemical sensor may include a glucose limiting membrane (GLM) that limits the amount of glucose and oxygen delivered to a glucose oxidase (GOx) layer of a working electrode of the sensor to ensure that the reactions are glucose limited. The GOx layer or another active enzyme layer on the working electrode of the sensor may break down glucose and oxygen into gluconic acid and hydrogen peroxide. The generated peroxide molecules may interact with the working electrode to break down hydrogen peroxide into two hydrogen ions, oxygen, and two electrons at the surface of the working electrode, when a voltage signal is supplied to the working electrode. The electrical charges may be forced to move between electrodes (e.g., between the working electrode and counter electrode), thereby generating a sensor current signal (Isig) that can be measured by sensor electronics. Other signals such as the counter voltage (Vcntr, the voltage potential difference between the counter electrode and the working electrode), electrochemical impedance spectroscopy (EIS) at different frequencies, and the like, may also be measured. The signals measured using the sensor, including the Isig, Vcntr, and EIS, may be processed (e.g., filtered or transformed) to generate some other signals or parameters, such as filtered Isig signals, real and imaginary impedance at various frequencies, and the like. These signals and/or the processed parameters may be used in one or more sensor glucose (SG) models (e.g., machine learning models or mathematical models) to determine SG values that may be estimations of the blood glucose (BG) levels of the patient.

As persons skilled in the art would understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of patient 101. In some embodiments, the electrical biosignal may include portions of a potential change produced by the heart of patient 101 over time (e.g., recorded as an electrocardiogram) that may be indicative of a glucose level of patient 101. An optical sensor may be configured to, for example, respond to the interaction or binding of a biological marker to a substrate by generating an electrical signal based on change in luminance of the substrate. In one example, the substrate may include a material selected to fluoresce in response to contact with a selected biomarker, such as glucose. The fluorescence may be proportional to a concentration of the selected biomarker.

In some embodiments, monitoring device 104 may include other types of sensors that may be worn, carried, or coupled to patient 101 to measure activity of patient 101 that may influence the glucose levels or glycemic response of patient 101. As an example, the sensors may include an acceleration sensor configured to detect an acceleration of patient 101 or a portion of the patient 101, such as the person's hands or feet, the position changes of which may be associated with an activity of patient 101. For example, the acceleration or movement (or lack thereof) of the body portion of patient 101 may be indicative of exercise, sleep, or food/beverage consumption activity of patient 101, which may influence the glycemic response of patient 101. In some embodiments, the sensors may measure heart rate and/or body temperature, which may indicate an amount of physical exertion experienced by patient 101. In some embodiments, the sensors may include a Global Positioning System (GPS) receiver which may detect GPS signals to determine a location of patient 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor may be referred to herein as a "sensor signal." As used herein, the term "sensed data" may mean and include the information represented by a sensor signal or by a pre-processed sensor signal. In some embodiments, sensed data may include glucose levels of patient 101, acceleration of a part of patient 101, heart rate of patient 101, temperature of patient 101, and/or geolocation (e.g., GPS location) of patient 101, among other things. Monitoring device 104 may communicate sensed data to delivery device 102 via communication link 112 and/or to computing device 106 via communication link 114. Use of sensed data by delivery device 102 and/or by computing device 106 is described in more detail below.

In some embodiments, monitoring device 104 may include components and/or circuitry configured to pre-process sensor signals. Pre-processing may include, for example, amplification, filtering, attenuation, scaling, isolation, normalization, transformation, sampling, and/or analog-to-digital conversion, among other things. In some embodiments, monitoring device 104 may host an App for process the sensor signals. In some embodiments, monitoring device 104 may include a wired or wireless transceiver as described above for transmitting the sensor signals or receiving commands or instructions. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, monitoring device 104 may not perform pre-processing.

Computing device 106 may provide processing capabilities and may be implemented in various ways. In some embodiments, computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of delivery device 102. In some embodiments, computing device 106 may be processing circuitry that may be integrated with another device, such as delivery device 102. In some embodiments, computing device 106 may be secured to patient 101 (e.g., to the body or clothing of patient 101), may be at least partially implanted into the body of patient 101, and/or may be held by patient 101.

For each of the embodiments of computing device 106, computing device 106 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Some aspects of delivery device 102, monitoring device 104, and computing device 106 have been described above. One or more of devices 102-106 may include a user interface (not shown) that presents information to patient 101 and/or receives information from patient 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify patient 101 of sensed data (e.g., glucose level) and/or insulin delivery data (e.g., rates of historic, current, or future insulin delivery) and may present alerts to patient 101. In some embodiments, a user interface may receive inputs from patient 101, which may include, for example, a requested change in insulin delivery setting and/or a meal indication, among other things. The descriptions and embodiments above regarding user interfaces are merely provided as examples, and other types and other uses of user interfaces are contemplated to be within the scope of the present disclosure.

In one specific example, the communications between devices 102-106 and cooperation between devices 102-106 may be used for insulin delivery. As depicted in FIG. 1 and as described above, devices 102-106 may communicate with each other via communication links 112-116. In some embodiments, computing device 106 may control operations of delivery device 102 and/or monitoring device 104. For example, computing device 106 may generate one or more signals (e.g., a command signal) that cause delivery device 102 to deliver insulin to patient 101, for example, as a basal dosage and/or a bolus dosage. In some embodiments, computing device 106 may receive data associated with insulin delivery (e.g., insulin delivery data) from delivery device 102 and/or receive sensed data (e.g., glucose levels) from monitoring device 104, and may perform computations based on the insulin delivery data, the sensed data, and/or other data to control delivery device 102. Insulin delivery data may include, but is not limited to, the type of insulin being delivered, historical insulin delivery rates and/or amounts, current insulin delivery rate and/or amount, insulin delivery time, and/or user inputs affecting insulin delivery. As persons skilled in the art will understand, in a closed-loop operating mode, computing device 106 may communicate dosage commands to delivery device 102 based on, for example, a difference between a current glucose level in the body of patient 101 (e.g., received from monitoring device 104) and a target glucose level (e.g., determined by computing device 106 or set on delivery device 102). The dosage commands may indicate an amount of insulin to be delivered and/or a rate (or time) of insulin delivery, and may regulate the current glucose level toward the target glucose level.

Remote/cloud computing system 108 may be any proprietary remote/cloud computing system or a commercial cloud computing system including one or more server computing devices. Remote/cloud computing system 108 may provide alternative or additional computing resources as needed when the computing resources of a client computing device (e.g., computing device 106) are not sufficient. Computing device 106 and remote/cloud computing system 108 may communicate with each other through a communication link 118, which may traverse one or more communication networks (not shown). The communication networks may include, for example, an Ethernet network, a Wi-Fi network, a cellular network, a satellite network, an intranet, an extranet, the Internet, and/or the Internet backbone, among other types of networks. Persons skilled in the art will recognize implementations for remote/cloud computing system 108 and how to interface with such systems through various types of networks. For example, remote/cloud computing system 108 may include an array of processing circuitry and may execute machine-readable instructions. Such implementations, interfaces, and networks are contemplated to be within the scope of the present disclosure.

In some embodiments, remote/cloud computing system 108 may make a therapy determination (e.g., an insulin amount or adjusted insulin amount), and may communicate the therapy to delivery device 102 via computing device 106. In some embodiments, computing device 106 may make the therapy determination and communicate it to delivery device 102. In some embodiments, monitoring device 104 may make the therapy determination and communicate it to delivery device 102 either directly or through an intermediary such as computing device 106.

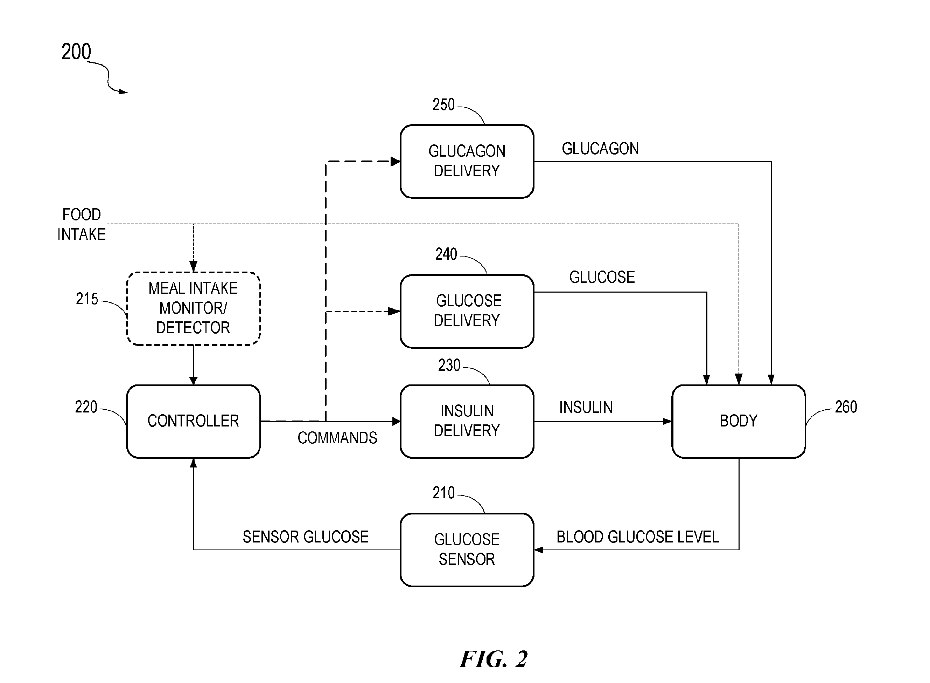

**FIG. 2** is a block diagram of an example of a glucose control system 200 according to certain embodiments. In the illustrated example, glucose control system 200 may include a glucose sensor subsystem 210, a controller 220, an insulin delivery subsystem 230, a glucose delivery subsystem 240, and a glucagon delivery subsystem 250. Glucose sensor subsystem 210 may generate sensor glucose (SG) signals (e.g., SG levels) that may be the estimations of blood glucose levels in a body 260, and may provide the SG signals to controller 220. Controller 220 may receive the SG signals and generate commands to insulin delivery subsystem 230, and, in some implementations, glucose delivery subsystem 240 and/or glucagon delivery subsystem 250. Insulin delivery subsystem 230 may receive commands from controller 220 and deliver insulin to body 260 according to the commands. In some embodiments, glucose delivery subsystem 240 may receive commands from controller 220 and provide glucose into body 260 according to the commands. In some embodiments, glucagon delivery subsystem 250 may receive commands from controller 220 and deliver glucagon into body 260 according to the commands.

In some implementations, glucose sensor subsystem 210 may include a glucose sensor, sensor electronics configured to generate SG signals, a sensor communication system configured to send the SG signals to controller 220, and a housing for the sensor electronics and the sensor communication system. The glucose sensor may measure blood glucose levels, for example, directly from a blood stream, or indirectly via interstitial fluid using a subcutaneous sensor as described in more detail below.

Controller 220 may include electrical components and software to generate commands for insulin delivery subsystem 230, glucose delivery subsystem 240, and/or glucagon delivery subsystem 250. Controller 220 may include a controller communication system to receive the sensor signal and provide the commands to insulin delivery subsystem 230, glucose delivery subsystem 240, and/or glucagon delivery subsystem 250. In some implementations, controller 220 may implement a glucose calculator. In some implementations, controller 220 may include a user interface and/or operator interface (not shown) comprising a data input device and/or a data output device. Such a data output device may, for example, generate signals to initiate an alarm and/or include a display or printer for showing status of controller 220 and/or a patient's vital indicators. Such a data input device may include dials, buttons, pointing devices, manual switches, alphanumeric keys, a touch-sensitive display, combinations thereof, and/or the like for receiving user and/or operator inputs. Such a data input device may be used for scheduling and/or initiating insulin bolus injections for meals, for example. It should be understood, however, that these are merely examples of input and output devices that may be a part of an operator and/or user interface and that claimed subject matter is not limited in these respects.

Insulin delivery subsystem 230 may include, for example, an infusion device and/or an infusion tube to infuse insulin into body 260. Similarly, glucose delivery subsystem 240 may include, for example, an infusion device and/or an infusion tube to infuse glucose into body 260. Likewise, glucagon delivery subsystem 250 may include, for example, an infusion device and/or an infusion tube to infuse glucagon into body 260. In some embodiments, the insulin, glucagon, and/or glucose may be infused into body 260 using a shared delivery system and/or infusion tube. In some embodiments, the insulin, glucagon, and/or glucose may be infused using an intravenous system for providing fluids to a patient (e.g., in a hospital or other medical environment). It should be understood, however, that certain example embodiments may include an insulin delivery subsystem 230 without a glucagon delivery subsystem 250 and/or without a glucose delivery subsystem 240. In some embodiments, each of insulin delivery subsystem 230, glucose delivery subsystem 240, and glucagon delivery subsystem 250 may include infusion electrical components to activate an infusion motor according to the commands from controller 220, an infusion communication system to receive commands from controller 220, and a delivery subsystem housing.

In some embodiments, controller 220 may be housed in a delivery subsystem housing, and an infusion communication system may comprise an electrical trace or a wire that carries the commands from controller 220 to the delivery subsystem. In some embodiments, controller 220 may be housed in a sensor system housing, and a sensor communication system may comprise an electrical trace or a wire that carries the sensor signal from sensor electrical components to controller electrical components. In some embodiments, controller 220 may have its own housing or may be included in a supplemental device. In some embodiments, controller 220 may be colocated with a delivery subsystem and a sensor system within a single housing. In some embodiments, a sensor, a controller, and/or infusion communication systems may utilize a cable; a wire; a fiber optic line; RF, IR, or ultrasonic transmitters and receivers; combinations thereof; and/or the like instead of electrical traces, just to name a few examples.

In some embodiments, glucose control system 200 may also include a meal intake monitoring subsystem 215. Meal intake monitoring subsystem 215 may be used to log the amount of user food intake, or may automatically detect the amount of user food intake. For example, in some implementations, the user may enter the food items and/or the estimated amount of carbohydrates in a meal at the meal time. In some implementations, meal intake monitoring subsystem 215 may include sensors (e.g., cameras or accelerators) that may automatically detect a meal event, the food items in a meal, and/or the estimated amount of carbohydrates in the meal. The estimated amount of carbohydrates in the meal may be sent to controller 220, which may determine an appropriate amount of meal bolus and generate a command for insulin delivery subsystem 230 to deliver the meal bolus. In some implementations, meal intake monitoring subsystem 215 may not be used in glucose control system 200, and the dosage for the meal bolus may be determined based on the measured glucose level.

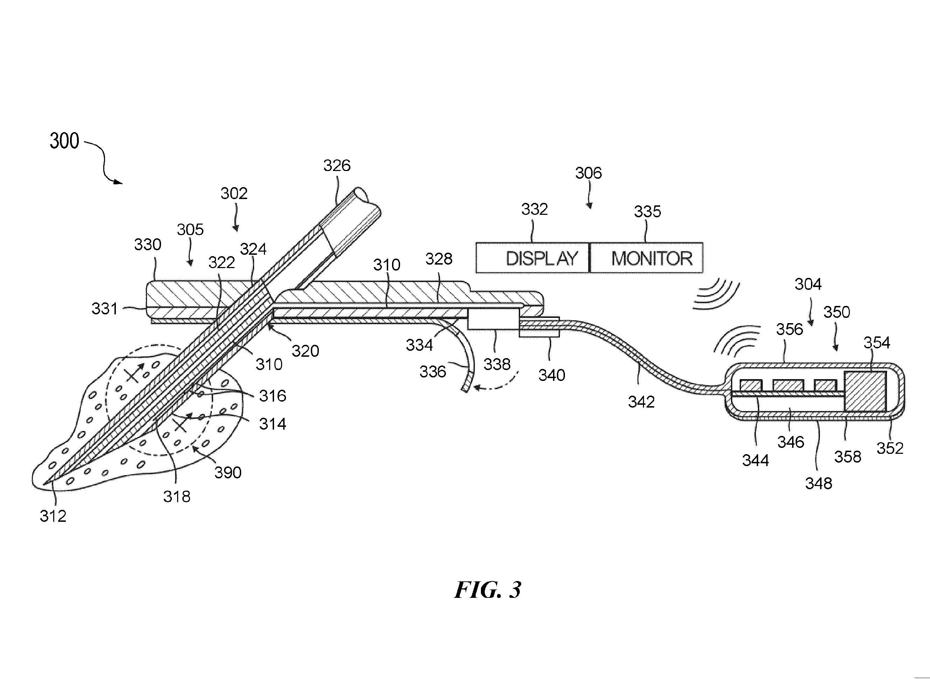

**FIG. 3** is a perspective view of an example of a CGM system 300 according to certain embodiments. In the illustrated example, CGM system 300 may include a subcutaneous sensor set 302 provided for subcutaneous placement of an active portion of a flexible sensor 310, or the like, at a selected site in the body of a user. The subcutaneous or percutaneous portion of sensor set 302 includes a hollow, slotted insertion needle 326 having a sharpened tip 312, and a cannula 322. Sensor set 302 may facilitate accurate placement of flexible sensor 310 into the body of the user. Inside cannula 322 is a glucose sensing portion 318 of flexible sensor 310. Glucose sensing portion 318 includes one or more glucose sensor electrodes 316 that may be exposed to the user's bodily fluids 390, for example, through a window 314 formed in the cannula 322. Sensor electrodes 316 may include, for example, a counter electrode, a reference electrode, and one or more working electrodes.

The proximal part of flexible sensor 310 may be mounted in a mounting base 305 adapted for placement onto the skin of the user. In some embodiments, mounting base 305 can be a pad having an underside surface coated with a suitable pressure sensitive adhesive layer 334, with a peel-off paper strip 336 normally provided to cover and protect the adhesive layer 334, until sensor set 302 is ready for use. Mounting base 305 may include an upper layer 330 and a lower layer 331, with a connection portion 328 of flexible sensor 310 being sandwiched between upper layer 330 and lower layer 331. Connection portion 328 may include a forward section joined to glucose sensing portion 318 of flexible sensor 310, which may be folded angularly to extend downwardly through a bore 320 formed in lower layer 331 of mounting base 305. Optionally, adhesive layer 334 (or another portion of the apparatus in contact with *in vivo* tissue) may include an anti-inflammatory agent to reduce an inflammatory response and/or anti-bacterial agent to reduce the chance of infection. Insertion needle 326 may be adapted for slide-fit reception through a needle port 324 formed in upper layer 330 of mounting base 305 and through lower bore 320 in the lower layer 331 of mounting base 305. In some embodiments, insertion needle 326 may be withdrawn after insertion to leave glucose sensing portion 318 (including glucose sensor electrodes 316) and/or cannula 322 in place at the selected insertion site.

Flexible sensor 310 of subcutaneous sensor set 302 may be connected to a sensor electronics device 304, which may be referred to as a transmitter. For example, mounting base 305 may be designed so that glucose sensing portion 318 may be joined to a connection portion 328 that terminates in conductive contact pads, or the like. Connection portion 328 and the contact pads may be adapted for an electrical connection to sensor electronics device 304 for determining the glucose level of the user in response to signals from sensor electrodes 316. Connection portion 328 may be connected electrically to sensor electronics device 304 by a connector block 338. Sensor electronics device 304 may include, for example, a housing 350 that supports a printed circuit board 344, batteries 354, and an antenna 346. In some embodiments, housing 350 may include an upper case 356 and a lower case 352 that are sealed by, for example, ultrasonic welding, to form a waterproof (or resistant) seal to permit cleaning by immersion (or swabbing) with water, cleaners, alcohol or the like. In some embodiments, upper case 356 and lower case 352 may be formed from a medical grade plastic. In alternative embodiments, upper case 356 and lower case 352 may be connected together by other methods, such as snap fits, sealing rings, RTV (silicone sealant) and bonded together, or the like, or formed from other materials, such as metal, composites, ceramics, or the like. In other embodiments, the assembly may be potted in epoxy or other moldable materials that is compatible with the electronics and reasonably moisture resistant. In some embodiments, sensor electronics device 304 may be connected to flexible sensor 310 through connector block 338 and a connector 340, and may be mounted onto mounting base 305. In some embodiments, sensor electronics device 304 may be connected to flexible sensor 310 through connector block 338, connector 340, and a cable 342. In some embodiments, lower case 352 may have a bottom surface coated with a suitable pressure sensitive adhesive layer 358, with a peel-off paper strip 348 normally provided to cover and protect the adhesive layer 358, until sensor electronics device 304 is ready for use.

Batteries 354 may include chargeable or non-chargeable batteries. In some embodiments, batteries 354 may include three series silver oxide battery cells. In other examples, different battery chemistries may be utilized, such as lithium based chemistries, alkaline batteries, nickel metalhydride, or the like, and a different number of batteries may be used. Batteries 354 may provide power to sensor set 302 via sensor electronics device 304 through, for example, connector block 338, connector 340, and/or cable 342.

Sensor electronics device 304 may include a processor, a transceiver unit, signal processing/conditioning circuits, and memory devices on printed circuit board 344. The processor may be configured to receive current and/or voltage signals from sensor electrodes 316 of flexible sensor 310. Sensor electrodes 316 may generate sensor signals indicative of a concentration of analyte (e.g., glucose) being measured. In one example, the sensor signals may include a current measured at a working electrode that may include a glucose oxidase (GOx) enzyme for catalyzing a reaction with glucose. The sensor signals may also include voltage signals measured at a counter electrode of sensor electrodes 316, or between a working electrode and a counter electrode (or reference electrode). In some examples, other electrical parameters, such as capacitance, resistance, impedance, and the like, may also be measured using sensor electrodes 316 inserted into the body of the user.

An example of the impedance parameters measured using sensor electrodes 316 may include electrochemical impedance spectroscopy (EIS) values. EIS may provide additional information in the form of sensor impedance and impedance-related parameters at different frequencies. Moreover, for certain ranges of frequencies, impedance and/or impedance-related data may be substantially glucose independent. Such glucose independence may enable the use of a variety of EIS-based markers or indicators for not only producing a robust, highly reliable sensor glucose value (e.g., through fusion methodologies), but also assessing the condition, health, age, and efficiency of individual electrode(s) of the overall sensor substantially independent of the glucose-dependent current signal. In one example, the analysis of the glucose-independent impedance data may provide information about the efficiency of a glucose sensor with respect to how quickly it hydrates and is ready for data acquisition. In addition, glucose-independent impedance data (using, e.g., values for 1 kHz real impedance) may provide information on potential occlusion(s) that may exist on a sensor membrane surface, where the occlusion(s) may temporarily block passage of glucose into the sensor and thus cause the signal to dip. Furthermore, glucose-independent impedance data may provide information on loss of sensor sensitivity during extended wear, for example, using values of phase angle and/or imaginary impedance at 1 kHz and higher frequencies.

The processor of sensor electronics device 304 may receive the sensor signals and calibrate the sensor signals using a calibration function that may be determined, for example, using reference values. In some embodiments, parameters (e.g., calibration factor or sensor sensitivity) of the calibration function may be determined (e.g., using calibration data) or pre-determined and stored in the memory for use by the processor. For example, in some embodiments, the processor may implement or otherwise execute a calibration application module that calculates or otherwise determines calibration parameters based on the measurement values and references values (e.g., blood glucose values measured using, for example, a fingerstick blood glucose meter). Based on the sensor signals and the determined calibration function, the processor may estimate the blood glucose level of the user. In some embodiments, the processor may store the glucose measurements in the memory. The sensor measurement results may be displayed on a display of sensor electronics device 304 or may be sent to a receiver 306 for display.

The memory of sensor electronics device 304 may be any type of memory device and may be configured to store raw sensor signals from flexible sensor 310 and/or glucose measurements (e.g., SG values) produced by the processor, calibration parameters used to determine glucose measurements from sensor signals, or other data used and/or produced by the processor. The memory may further store software and/or firmware that is executable by the processor. In some embodiments, the memory may further include configuration information for configuring the operation of sensor electronics device 304.

The signal processing/conditioning circuits of sensor electronics device 304 may include one or more amplifiers, filters, current to voltage converters, integrators, analog-to-digital converters, potentiostat, and the like. The signal processing/conditioning circuits may filter the signals, apply a constant reference voltage level, amplify current or voltage signals, sample and convert analog current or voltage signals into digital data values, and the like.

The transceiver unit of sensor electronics device 304 may include a wireless transceiver that may send the glucose measurements determined by the processor and stored in the memory to receiver 306 using a suitable wireless communication protocol, such as Bluetooth (e.g., Bluetooth low energy (BLE)), Wi-Fi, WiMax, and the like. For example, the transceiver unit may send radio signals indicative of the glucose level at regular time periods (e.g., every 5 minutes) to provide real-time sensor glucose (SG) values. The transceiver unit may also receive data, such as configuration data and calibration data (e.g., reference glucose values), from receiver 306. In some embodiments, the transceiver unit of sensor electronics device 304 may send data to receiver 306 through a cable or wire.

Receiver 306 may include a monitor 335 for monitoring physiological characteristics readings and/or generating alert signals, and a display 332 for displaying physiological characteristics readings and/or alert signals. For example, SG values/graphs may be displayed on display 332 of receiver 306 so that a user can monitor the blood glucose level and administer insulin using an insulin pump. In various embodiments, receiver 306 may be implemented in a desktop computer, a pager, a television including communications capabilities, a laptop computer, a server, a network computer, a personal digital assistant (PDA), a smart phone, an infusion pump including a display, a glucose sensor including a display, a combined infusion pump/glucose sensor, and the like.

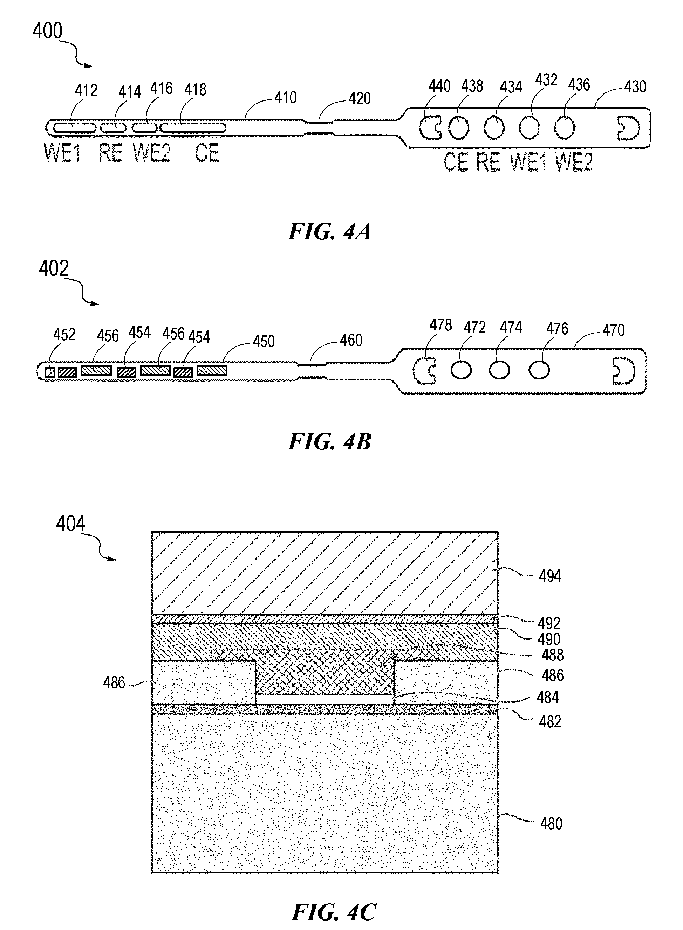

**FIG. 4A** illustrates an example of a glucose sensor 400 according to certain embodiments. Glucose sensor 400 may be an example of flexible sensor 310 described above. In the illustrated example, glucose sensor 400 may include a sensing portion 410 that includes one or more electrodes, such as a first working electrode (WE1) 412, a reference electrode (RE) 414, a second working electrode (WE2) 416, and a counter electrode (CE) 418, where the electrodes may be electrically isolated by an isolation layer. Sensing portion 410 may be an in vivo portion (also referred to as a distal end) that may be inserted into the subcutaneous layer of a user using a hollow needle, such as insertion needle 326. Glucose sensor 400 may include a bending region 420, which may be bent by, for example, up to about 90°, before or after insertion. Glucose sensor 400 may also include a contact pad region 430, which may be an ex vivo portion of glucose sensor 400. As illustrated, contact pad region 430 may include one or more contact pads electrically connected to the one or more electrodes in sensing portion 410 by conductive traces (not shown). The contact pads may include, for example, a contact pad 438 connected to counter electrode 418, a contact pad 434 connected to reference electrode 414, and two contact pads 432 and 436 connected to first working electrode 412 and second working electrode 416, respectively. The contact pads in contact pad region 430 of glucose sensor 400 may include, for example, a metal layer, and may be connected to one or more contact pads on a printed circuit board of a sensor electronics device. In the illustrated example, contact pad region 430 of glucose sensor 400 may also include a pair of guide holes 440 that may fit a pair of guide pins on the printed circuit board to align contact pads on contact pad region 430 with contact pads on the printed circuit board, such that voltage required for glucose sensing may be applied and sensor current proportional to interstitial glucose concentration may be measured by the sensor electronics on the printed circuit board.

FIG. 4B illustrates another example of a glucose sensor 402 according to certain embodiments. Glucose sensor 402 may be another example of flexible sensor 310 described above. In the illustrated example, glucose sensor 402 may include a sensing portion 450 that includes one or more electrodes, such as a reference electrode (RE) 452, one or more working electrode (WEs) 454, and one or more counter electrodes (CEs) 456, where the electrodes may be electrically isolated by an isolation layer. Sensing portion 450 may be an in vivo portion that may be inserted into the subcutaneous layer of a user using a hollow needle, such as insertion needle 326. Glucose sensor 402 may include a bending region 460, which may be bent by, for example, up to about 90°, before or after insertion. Glucose sensor 402 may also include a contact pad region 470, which may be an ex vivo portion of glucose sensor 402. As illustrated, contact pad region 470 may include one or more contact pads electrically connected to the one or more electrodes in sensing portion 450 by conductive traces (not shown). The contact pads may include, for example, a contact pad 472 connected to reference electrode 452, a contact pad 474 connected to working electrodes 454, and a contact pad 476 connected to counter electrodes 456. The contact pads in contact pad region 470 of glucose sensor 402 may include, for example, a metal layer, and may be connected to one or more contact pads on a printed circuit board of a sensor electronics device. In the illustrated example, contact pad region 470 of glucose sensor 402 may also include a pair of guide holes 478 that may fit a pair of guide pins on the printed circuit board to align contact pads on contact pad region 470 with contact pads on the printed circuit board, such that voltage required for glucose sensing may be applied and sensor current proportional to the interstitial glucose concentration may be measured by the sensor electronics on the printed circuit board.

FIG. 4C illustrates a cross-section of an example of a working electrode 404 of a glucose sensor according to certain embodiments. As illustrated, working electrode 404 may include a substrate or base layer 480 (e.g., including polyimide), which provides the foundation and rigidity of a flex substrate. An overlying plated metallic layer 482 (e.g., including chromium and/or gold) may be deposited on base layer 480. The chrome layer may promote adhesion between the polyimide and the gold layer, which enables the sensor to transfer charges when a bias voltage is applied. An electroplated layer 484 (e.g., including platinum) may be formed on metallic layer 482, e.g., in a trench formed in an insulating layer 486 (e.g., including polyimide). Insulating layer 486 may help to improve sensor integrity by providing electrical insulation between the working, counter, and reference electrodes, and may also serve as a supportive barrier for the electrodes and sensor flex chemistry. Electroplated layer 484 may be the active electrode for analyte (glucose) sensing, where glucose may be sensed at the working electrode through the oxidation of hydrogen peroxide to generate electrical charges. The working electrode may also be responsible for holding a bias potential $V_{set}$ applied by the sensor electronics. A glucose oxidase (GOx) layer 488 is formed on insulating layer 486, for example, by depositing a glucose oxidase solution (e.g., via slot coating) in the trench formed in insulating layer 486. A human serum albumin (HSA) layer 490 is formed overlying glucose oxidase layer 488. An adhesive promoter layer 492 may be used on HSA layer 490 to affix a glucose limiting membrane (GLM) layer 494 on working electrode 404. It is noted that FIG. 4C depicts a simplified representation of an example of a working electrode, and practical embodiments may include any number of additional and/or alternative layers (e.g., a high-density amine (HDA) layer, etc.). Accordingly, the subject matter described herein is not intended to be limited to the embodiment depicted in FIG. 4C. A reference electrode or counter electrode may have structures similar to the structure shown in FIG. 4C but may have some modification. For example, in some embodiments, a reference electrode may include an Ag layer, rather than a platinum layer, and may not include a glucose oxidase layer.

Glucose oxidase in GOx layer 488 may include the active enzyme of the sensor. In the presence of oxygen, GOx may break down glucose into gluconic acid and hydrogen peroxide. HSA layer 490 may break down hydrogen peroxide into water. Adhesive promoter layer 492 may include, for example, 3-aminopropyltriethoxysilane (APTES), which is a compound that provides a better adhering surface between the HSA layer 490 and GLM layer 494. GLM layer 494 may limit the amount of glucose and oxygen delivered to GOx layer 488 of working electrode 404 to ensure that the reactions are glucose limited. GOx layer 488 or another active enzyme layer on working electrode 404 may break down glucose and oxygen into gluconic acid and hydrogen peroxide. The generated peroxide molecules may interact with working electrode 404 (e.g., biased at about 0.2 V to about 0.7 V) to break down hydrogen peroxide into two hydrogen ions, oxygen, and two electrons at the surface of working electrode 404. The electrical charges may be forced to move between the electrodes, thereby generating a sensor current signal (Isig) that can be measured by sensor electronics. For example, when a GOx enzyme is used as a catalytic agent in the glucose sensor, an electrical current may flow from the counter electrode to the working electrode if there is oxygen in the vicinity of the enzyme and the sensor electrodes. Other signals such as the counter voltage (Vcntr, e.g., the voltage potential difference between the counter electrode and the working electrode), electrochemical impedance spectroscopy (EIS) signals at different frequencies, and the like, may also be measured.

In some embodiments, measurements of the parameters of the electrodes of the electrochemical glucose sensors described above may be performed during and/or after the fabrication processes. For example, an imaginary impedance of the working electrode (and the counter electrode) may be measured after a platinum electroplating process to form electroplated layer 484. GOx solution activity measurements may be performed during or after the GOx solution preparation process prior to deposition, and the thickness of GOx layer 488 may be measured after the slot coating and selective patterning processes over working electrode 404. The HSA concentration may be measured during or after the solution preparation process prior to coating HSA layer 490, and the thickness of GLM layer 494 may be measured after forming GLM layer 494. In some embodiments, these measurements may be performed using sacrificial or monitor instances of glucose sensors fabricated on a wafer. In some embodiments, additional fabrication process measurements such as surface roughness or other topographic measurements may be performed (e.g., via interferometry) for the electrodes during or after a fabrication process.

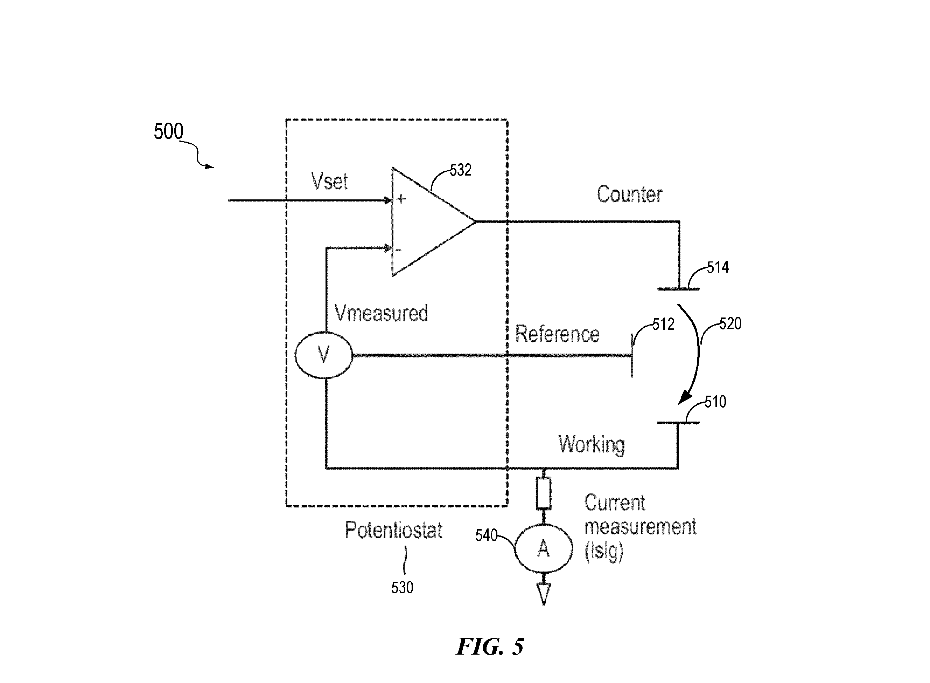

FIG. 5 shows a schematic of an example of a glucose sensor 500 according to certain embodiments. Glucose sensor 500 may include a potentiostat 530 electrically connected to the electrodes of glucose sensor 500. As illustrated, potentiostat 530 may include an operational amplifier (op amp) 532 (or other servo-controlled device) that includes two inputs $V_{set}$ and $V_{measured}$. $V_{measured}$ may be the measured voltage between a reference electrode 512 and a working electrode 510 of a sensor flex as described above. $V_{set}$ may be the desired voltage across working electrode 510 and reference electrode 512. The output of op amp 532 may be connected to a counter electrode 514 to apply a voltage on counter electrode 514. Op amp 532 may utilize feedback through the sensor electrodes to attempt to maintain a desired voltage between reference electrode 512 and working electrode 510 by adjusting the voltage at counter electrode 514. Counter electrode 514 may balance the chemical reaction occurred at working electrode 510. A current 520 may flow between counter electrode 514 and working electrode 510 due to the electrochemical reaction between glucose and the sensor chemistry of working electrode 510 as described above. The current may be indicative of the glucose level in the interstitial fluid of the user and may be measured a current meter 540 as the sensor current (Isig) or may be output to processing circuits of sensor electronics for measurement. Other signals such as the counter voltage (Vcntr, e.g., the voltage potential difference between the counter electrode and the working electrode), electrochemical impedance spectroscopy (EIS) values at different frequencies, and the like, may also be measured using sensor electronics of the glucose sensor. Based on these raw sensor signals measured, the blood glucose level of the user may be determined using one or more models.

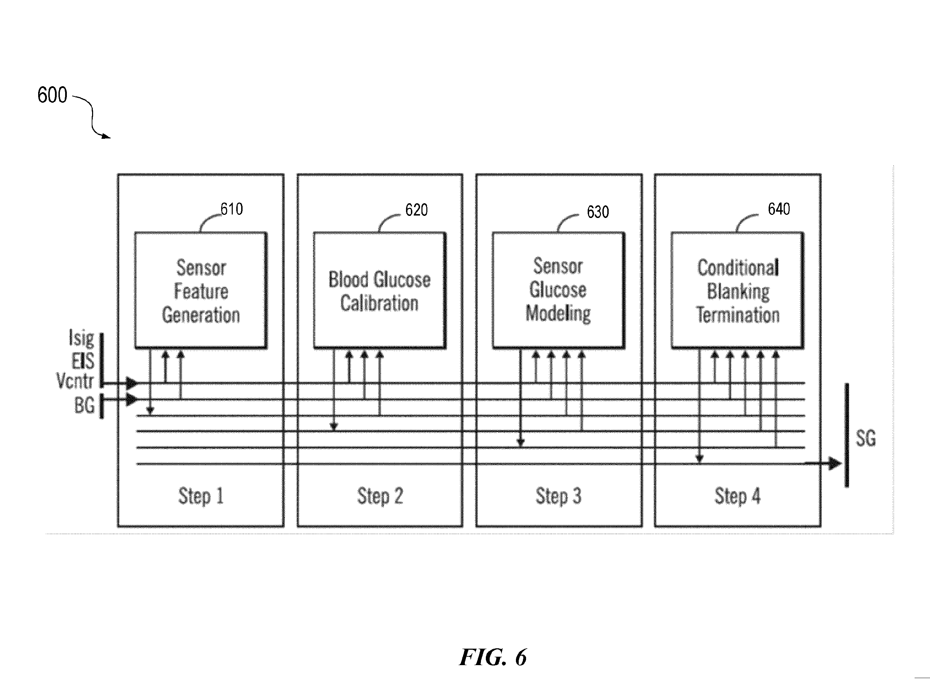

FIG. 6 includes a flow diagram 600 illustrating an example of a method of determining sensor glucose values using input data from a glucose sensor, in accordance with certain embodiments. In the example illustrated in FIG. 6, the method may include sensor feature generation at block 610, optional blood glucose calibration at block 620, sensor glucose modeling at block 630, and optional conditional blanking and termination at block 640. In some embodiments, sensor feature generation at block 610 may use raw interstitial current signals, electrochemical impedance spectroscopy signals, and counter voltage signals to extract input features used by downstream SG models (e.g., machine learning models). The optional blood glucose calibration at block 620 may include adjusting the input sensor features from block 610 based on calibration parameters determined using reference blood glucose values. Sensor glucose modeling at block 630 may include applying machine learning techniques to the input features to convert the input features into sensor glucose values. Conditional blanking and termination at block 640 may include applying various tests and logic to determine, for example, when to stop displaying sensor output signals or terminate the sensor to reduce the probability of displaying noisy or erroneous information to the user or receiving device. In some embodiments, terminating the sensor may include ceasing transmission of sensor data from the sensor device. In some embodiments, input data (*e.g.*, interstitially measured Isig, Vcntr, EIS, and/or BG values) may pass through blocks 610-640 and be transformed into SG values. Table 1 below shows an example of the inputs and outputs of each of the four blocks of flow diagram 600. In some embodiments, the processes of flow diagram 600 may be implemented using one or more machine learning models.

Table 1. Information Transfer in Flow Diagram 600

| Information | Block 610 | Block 620 | Block 630 | Block 640 |
|---|---|---|---|---|
| Isig, Vcntr, EIS, BG | Input | N/A | N/A | Input |
| Base and Derivative Sensor Features w/o Calibration | Output | Input | Input | Input |
| Base and Derivative Sensor Features after BG Calibration | N/A | Output | Input | Input |
| Initial Estimates of SG Values | N/A | N/A | Output | Input |
| Final Estimates of SG Values | N/A | N/A | N/A | Output |

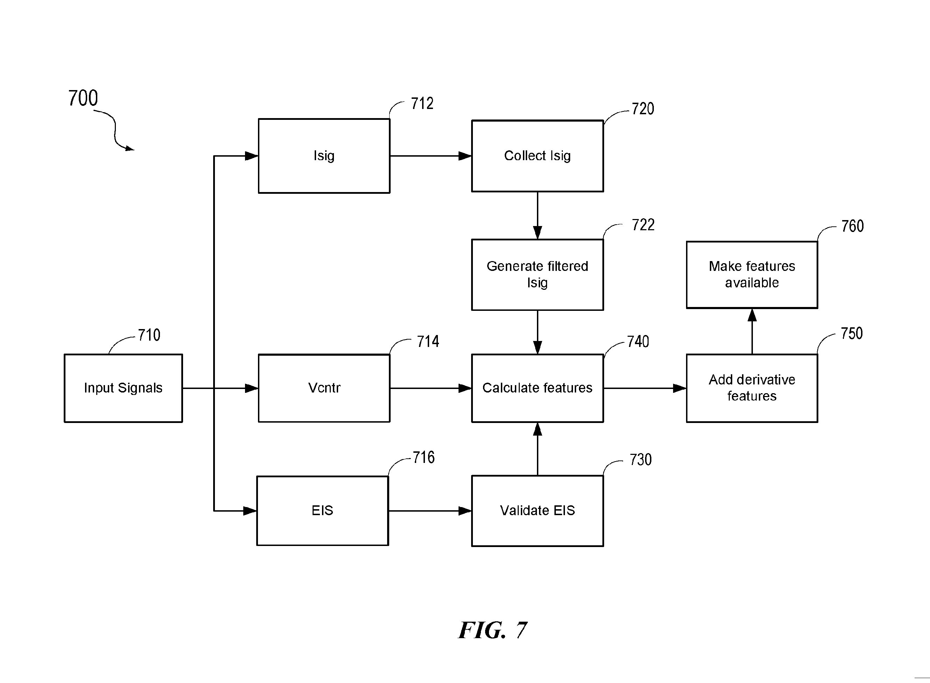

FIG. 7 illustrates an example of a sensor feature generator 700 according to certain embodiments. Sensor feature generator 700 may be used to perform the operations of sensor feature generation at block 610 of FIG. 6. In the illustrated example, input signals 710 to sensor feature generator 700 may include Isig signals 712 (*e.g.*, at an interval about 1 minute), Vcntr signals 714 (*e.g.*, at an interval about 5 minutes), and EIS signals 716 (*e.g.*, at an interval about 15-30 minutes). Isig signals 712 may be collected over, for example, 5 or more minutes at block 720, and the collected Isig signals 712 may be used to generate filtered Isig signals 722 (over a 5-minute time window). For example, the most recent 8 minutes of Isig signals 712 may be filtered using, for example, a seventh order finite impulse response (FIR) filter to create a filtered Isig signal 722. In some implementations, any computed filtered Isig signal 722 that is invalid may be discarded, for example, if a value of the recent 8 minutes of Isig signals 712 is larger than twice of the mean value of the 8 minutes of Isig signals 712 or lower than a half of the mean value of the 8 minutes of Isig signals 712. In some embodiments, artifact detection may be performed to identify large and small drops in Isig signals 712 or filtered Isig signal 722. EIS signals 716 may include measurements taken using signals of various frequencies, such as a set of frequencies between about 0.1 Hz and about 8 KHz. EIS signals 716 may be validated to generate a validated EIS signal 730. In some embodiments, validated EIS signal 730 may be converted to 5-minute sampling using zero-order hold. In some embodiments, the frequency of measuring the signals can change depending on the hardware design. For example, Vcntr may be measured at 1-minute and EIS may be measured more frequently, depending on factors such as battery life and memory size limitations.

The filtered Isig signal 722, validated EIS signal 730, and Vcntr signals 714 may be used to generate 5-minute features 740, where the features for each 5-minute time period may be included in a respective data packet. Derivative features 750 may be added to the data packet based on 5-minute features 740 in the current data packet and previous 5-minute features 740 in previous data packets. Final features 760 including the 5-minute features 740 and the derivative features 750 may include, for example, filtered Isig signal 722, previous filtered Isig signals, an accumulative average of Isig signals, a moving average of Isig signals, filtered Isig signals, Vcntr signal 714 of current data packet, Vcntr signals of previous data packets, sensor age, real and imaginary components of EIS signals at various frequencies, changes of the real and imaginary components of EIS signals at various frequencies, and the like. In some embodiments, sensor feature generator 700 may process the Isig signals, Vcntr signals, and EIS signals based on a different time interval (*e.g.*, shorter or longer than 5 minute). A data packet including all or some of 5-minute features 740 and derivative features 750 may be used for optional blood glucose calibration at block 620, sensor glucose modeling at block 630, and optional conditional blanking and termination at block 640.

Due to manufacturing variances and aging of the glucose sensors, some glucose sensors may not provide accurate readings based on measured signals and derived signal features. As such, the glucose sensors may need to be occasionally or periodically calibrated in vitro and/or in vivo to improve the accuracy and/or reliability of the glucose sensors. As shown in the example of FIG. 6, the sensor signals may be calibrated at block 620 using calibration functions determined using known good blood glucose values, such as fingerstick measurements from blood glucose meters that measures the blood glucose level in the capillaries. For example, a calibration ratio CR (or a calibration factor) may be determined according to CR=BG/(Isig + offset). The input signals, such as Isig signals, may then be calibrated using the calibration ratio and/or the offset. In some embodiments, a weighted calibration ratio may be determined based on previous calibration ratios and time of the previous calibrations and applied to all subsequent data packets until next calibration is performed.

At block 630, sensor glucose values may be derived from the generated features using one or more SG models. The SG models may include, for example, functions, equations, machine learning models, or other translation models that may map the generated features, Isig signals, Vcntr signals, and/or EIS signals to a sensor glucose (SG) value indicative of an estimated blood glucose level. For example, the features generated at block 610 and/or block 620 may be input to the one or more SG models as a feature vector, and the SG value may be determined as the dot product of the feature vector and a coefficient vector with corresponding coefficients (e.g., weights). For example, an SG model may be represented by:

$$SG = f(Age, Isig, Vcntr, EIS).$$

In a simplified example, SG values may be calculated according to:

$$SG = \alpha \times Age + \beta \times Isig + \gamma \times Vcntr + \delta \times EIS + \Delta,$$

where $\alpha$, $\beta$, $\gamma$, and $\delta$ may be coefficients associated with the age, Isig, Vcntr, and EIS, and $\Delta$ is an offset value. In some embodiments, at least one of Isig, Vcntr, or EIS may include a vector, and at least one of $\beta$, $\gamma$, or $\delta$ may be a coefficient vector. In some embodiments, at least one of $\alpha$, $\beta$, $\gamma$, and/or $\delta$ may be a function of time (e.g., the sensor age). In some embodiments, different SG models may be used for different input parameter ranges, such as different ranges of the age, Vcntr, or other sensor measurement data. One model may be selected from the one or more models based on the ranges that the input parameters fall in.

In embodiments where the one or more models include machine learning models, the machine learning models may include neural networks or other machine learning models. For example, the machine learning models may include neural networks that include a large number of nodes (neurons) arranged in one or more layers. Each node of a neural network may be connected with one or more other nodes of the neural network, where the connections may have associated coefficients or weights. In some embodiments, each individual node may implement a multiplication and accumulation function that combines the weighted values of all its inputs based on the associated weights. In some embodiments, each connection (or the node) may have a threshold function such that only signals that surpass the threshold may propagate to other nodes. The neural network may be trained or self-learning using training samples, rather than being explicitly programmed. In some embodiments, the neural network may include multiple layers each including multiple nodes, where input signals may traverse the multiple layers to generate a final output. In some embodiments, techniques such as back propagation techniques may be utilized to train the neural network, such as determining the coefficients or weight associated with the connections between nodes. Data for training the machine learning model may include, for example, sensor data (e.g., Isig, Vcntr, EIS, etc.) and corresponding known SG or BG values.

In some embodiments, the machine learning model may receive a multi-dimensional feature vector as inputs. For example, the inputs to the machine learning model may include features such as EIS features, Isig trending features, Isig rate of change features, previous Isig values, previous Vcntr values, and the like. Some examples of the input features and models used for determining SG values can be found in, for example, U.S. Pat. Appl. No. 16/773,422, entitled "METHODS, SYSTEMS, AND DEVICES FOR CONTINUOUS GLUCOSE MONITORING," which is herein incorporated by reference in its entirety. The outputs of the machine learning model may depend on combinations of the input features. In some embodiments, the one or more models may include a plurality (e.g., N) of models (e.g., functions, equations, machine learning models, etc.). The models may use the same or different input features and/or or may weigh the input features differently using different weights. In some embodiments, one or more models may be selected based on the input data and used to determine an SG value. For example, in some embodiments, outputs of two or more models selected from the plurality of models may be combined to generate a final SG value.

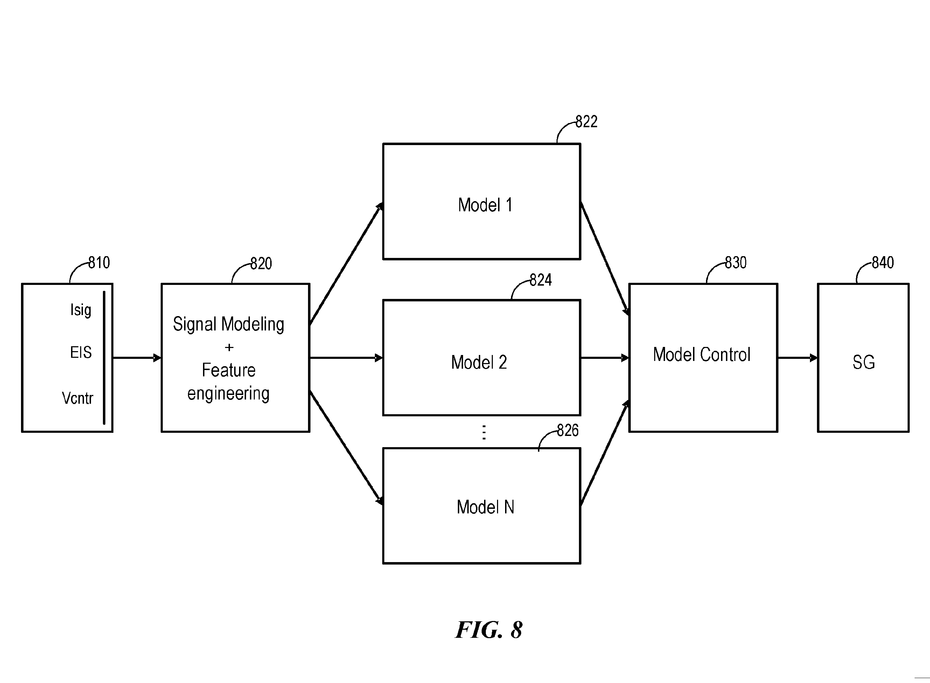

FIG. 8 illustrates an example of determining a sensor glucose value using one or more models according to certain embodiments. As shown in FIG. 8, inputs 810 to the one or more models may include Isig, EIS, Vcntr, or other input signals. In some embodiments, inputs 810 may additionally include prior modeling (e.g., trending) information. In the illustrated example, a signal modeling and feature engineering engine 820 may include N models, such as models 822, 824, ... , and 826, that may be trained or otherwise determined for various combinations of inputs or scenarios. As described above, the models may include machine learning models and may be trained to use different inputs or weigh the inputs differently. A model controller 830 may perform selections, averaging, ranking, weighting, or other processing of sensor glucose values generated by models 822, 824, ... , and 826. For example, model controller 830 may select a model (e.g., from models 822, 824, ... , and 826) based on inputs 810 or other information, such as age of the sensor, to determine an SG value. In some embodiments, model controller 830 may average the outputs of two or more models of models 822, 824, ... , and 826 to generate a final sensor glucose value 840. In some embodiments, model controller 830 may take the average, weighted average, median, maximum, minimum, and the like of the outputs of a subset of models 822, 824, ... , and 826. In some embodiments, model controller 830 may rank or weigh models 822, 824, ... , and 826, and may select the high ranking model(s). In some embodiments, a confidence score associated with the output of each of models 822, 824, ... , and 826 may be generated to indicate the confidence level that the output is correct, and model controller 830 may adjust the model outputs to favor one or more models associated with the highest levels of confidence. The final SG value 840 may be displayed to the user or may be sent to an insulin pump or an insulin calculator. In some embodiments, a confidence score associated with SG value 840 may be generated to indicate the confidence level that the final SG value 840 is correct.

At block 640, conditional blanking and termination may be performed on the SG values generated by the SG models. For example, some SG values generated by the one or more SG models may be blanked and not sent or displayed to the user if the SG values are determined to be outliers or otherwise erroneous. In some embodiments, the conditional blanking and termination at block 640 may be performed by one or more models such as machine learning models used at block 630 or other machine learning models. For example, a machine learning model may classify the SG values as being outliers/not outliers, having large negative bias/large positive bias/nominal accuracy, having poor accuracy/ intermediate accuracy/good accuracy, and the like. The SG values may be blanked based on the outputs from the machine learning model. For example, if the output of the machine learning model indicates that an SG value is not an outlier (e.g., within a certain threshold range), the SG value may not be blanked. Otherwise, the SG value may be blanked. Similarly, if the output of the machine learning model indicates that an SG value has an intermediate, normal, or good accuracy, the SG value may not be blanked. Otherwise, the SG value may be blanked. In some embodiments, a sensor may be terminated (e.g., stop calculating or transmitting any sensor data from that sensor and/or stop displaying any sensor data from that sensor) when, for example, a large number of outliers are generated using sensor measurement signals from the sensor.

As described above, sensor properties associated with a glucose sensor, such as the age (or time of use or wear time), battery level, calibration accuracy, sensing environment (e.g., oxygen level, temperature, moisture, and pH value, etc.), physiological dynamics, manufacturing variability, and other properties, may affect the enzymatic glucose level measurement results in complex ways that may be difficult to model. Therefore, many CGM sensors may have suboptimal accuracy and may exhibit large variance in the measurement results. The lower than desired performance of the CGM sensors may be at least partially attributed to sensor-to-sensor manufacturing variability, degradation of the sensor performance over time, complexity of modeling the in vivo behavior of the CGM sensors (e.g., it may be difficult to model the CGM sensor using a same model), and the like.

To achieve the desired accuracy and reliability and reduce the impact of noise and other spurious signals, CGM sensors may need to be periodically or occasionally calibrated in vitro and/or in vivo to improve the accuracy and/or reliability of the glucose sensors. For example, the sensor data may need to be calibrated using a known good blood glucose value, often obtained via a so-called "fingerstick measurement" using a blood glucose meter that measures the blood glucose level in the capillaries. Performing such calibration measurements may increase the patient burden and perceived complexity, and can be inconvenient, uncomfortable, or otherwise disfavored by patients. Moreover, ISF glucose measurements may lag behind the blood glucose measurements due to the time it takes glucose to diffuse from the capillary to the interstitial space, which may require certain signal processing (e.g., filtering) or other techniques to compensate for the physiological lag. Additionally, various factors can lead to transient changes in the sensor output, which may influence the accuracy of the calibration and the measurement. Degradation of sensor performance over time, which may also vary across sensors due to, for example, manufacture variations between the sensors, may further compound these challenges.

According to certain embodiments disclosed herein, some in vivo characteristics (e.g., in vivo sensor sensitivity and intercept) of a glucose sensor may be predicted before sensor insertion and may be used in one or more SG models to estimate SG values based additionally on other input data, such as Isig, Vcntr, EIS, age, and features derived therefrom Isig, Vcntr, EIS, and/or age. For example, the in vivo characteristics of a glucose sensor may be predicted using in vitro characteristics of the glucose sensor and an in vitro to in vivo translation model, where the in vitro characteristics of the glucose sensor may be predicted using fabrication process measurement data associated with the glucose sensor or may be measured in vitro. In some embodiments, the in vivo sensor characteristics may be predicted directly using the fabrication process measurement data associated with the glucose sensor. The in vivo characteristics may be predicted as a function of time (e.g., the age of the glucose sensor). In this way, at least some of the manufacturing variability and degradation of the glucose sensor can be accounted for in the SG calculation, and thus the SG values may be more accurately determined from raw sensor measurement data (e.g., Isig, Vcntr, EIS, and age).

In one example, fabrication process measurement data associated with an electrochemical glucose sensor, such as, for example, process parameters, thicknesses of various layers of the sensors, material compositions and/or concentrations, certain impedance and/or capacitance measurements, and the like, may be obtained during and/or after the fabrication, and may be used to determine in vitro characteristics of the electrochemical glucose sensor, such as in vitro sensitivity ($Sensitivity_{in\_vitro}$) and intercept ($Intercept_{in\_vitro}$) that may characterize the relationship between an in vitro senor current $Isig_{in\_vitro}$ and an in vitro SG value $SG_{in\_vitro}$ approximately according to:

$$SG_{in\_vitro} = Isig_{in\_vitro} / Sensitivity_{in\_vitro} + Intercept_{in\_vitro}$$

In vivo characteristics (e.g., in vivo sensitivity $Sensitivity_{in\_vivo}$ and intercept $Intercept_{in\_vivo}$) of the electrochemical glucose sensor may then be determined using the in vitro characteristics of the electrochemical glucose sensor determined using the fabrication process measurement data and one or more translation models according to, for example:

$$Sensitivity_{in\_vivo} = f(Sensitivity_{in\_vitro}),$$

and

$$Intercept_{in\_vivo} = f(Intercept_{in\_vitro})$$

In some embodiments, the in vitro to in vivo translation models may use other in vitro characteristics as inputs. For example, the in vivo sensitivity may be a function of the in vitro sensitivity and other parameters, such as the in vitro intercept. Both the in vitro characteristics and the in vivo characteristics of the electrochemical glucose sensor may vary with the age of the electrochemical glucose sensor and thus may be time-variant (a function of time).

Based on the in vivo characteristics (e.g., in vivo sensitivity and in vivo intercept) of the electrochemical glucose sensor, SG values may be determined using one or more SG models that utilize the in vivo characteristics as part of the inputs in addition to the sensor measurement data. The one or more SG models may include, for example, one or more machine learning models, equations, and the like. For example, one SG model may be represented by:

$$SG = f(Age, Isig, Vcntr, EIS, Sensitivity_{in\_vivo}, Intercept_{in\_vivo}).$$

In a simplified example, SG values may be determined according to:

$$SG = \alpha \times Age + \beta \times \frac{Isig}{Sensitvity_{in\_vivo}} + \gamma \times Vcntr + \delta \times EIS + \theta \times Intercept_{in\_vivo},$$

where $\alpha$, $\beta$, $\gamma$, $\delta$, and $\theta$ are coefficients that may or may not be time-variant and may include individual values, vectors, or matrixes. In some embodiments, the equation above may include an offset value. In some embodiments, at least one of Isig, Vcntr, EIS, $Sensitvity_{in\_vivo}$, or $\theta \times Intercept_{in\_vivo}$ may include a vector. In some embodiments, $\alpha$, $\beta$, $\gamma$, $\delta$, and $\theta$ may have different values for different input parameter ranges (e.g., different age and/or Vcntr ranges). As a result, the effect of fabrication process variability and sensor degradation over time on the sensor measurement data may be at least partially accounted for by using the time-variant in vivo characteristics of the sensor in the SG models. Therefore, the determined SG values may be more accurate estimations of the blood glucose levels.

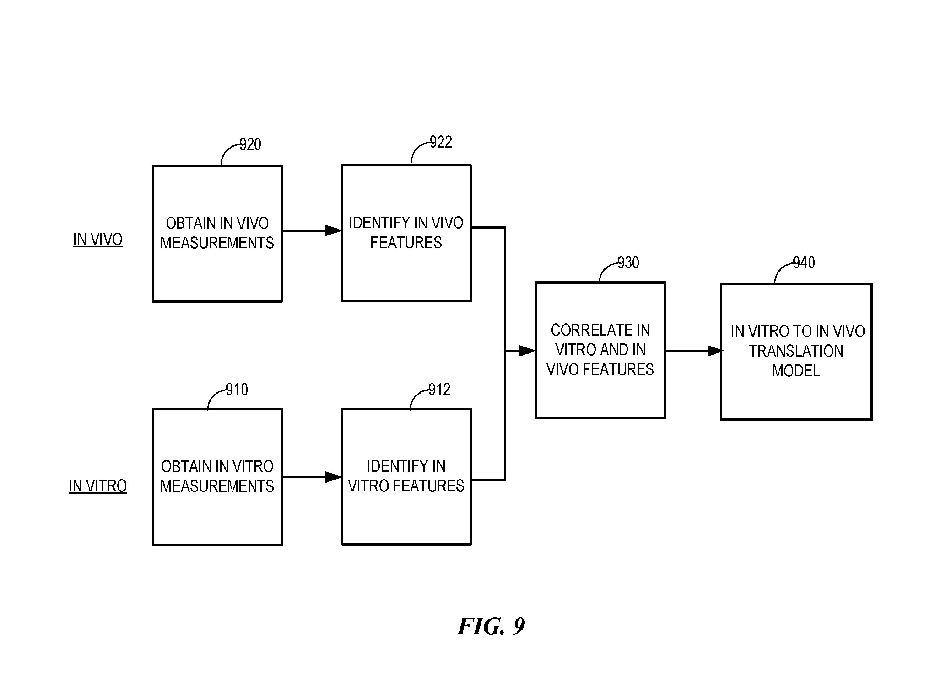

**FIG. 9** illustrates an example of determining a translation model for mapping in vitro characteristics of a CGM sensor to in vivo characteristics of the CGM sensor according to certain embodiments. The translation model may be determined based on sensor signals measured in vitro and sensor signals measured in vivo (e.g., clinical data). As illustrated, in vitro measurements and in vivo measurements may be performed at blocks 910 and 920, respectively. The in vitro and in vivo measurements may be obtained from, for example, in vitro testing and clinical feasibility studies, respectively. In one example, the in vitro testing may be performed by inserting glucose sensors into a custom made flow cell including sugar analyte, and measuring the glucose concentration in the flow cell by the glucose sensors. In some embodiments, the in vitro measurements may be performed at different time (e.g., at different ages after the first use) by each glucose sensor, and the in vivo measurements may also be performed at different time (e.g., at different ages after the first use) by each glucose sensor.

At block 912, in vitro characteristics, such as in vitro sensitivity and in vitro intercept, may be determined for the sensors based on the in vitro measurements and the relationship between the in vitro characteristics and the in vitro measurement results (e.g., *SG = Isig / Sensitivity + Intercept*). At block 922, in vivo characteristics, such as in vivo sensitivity and in vivo intercept, may be determined for the sensors based on the in vivo measurements and the relationship between the in vivo characteristics and the in vivo measurement results. At block 930, the in vitro characteristics and in vivo characteristics may be correlated, for example, using clustering techniques, regression techniques, or other machine learning techniques, to determine a translation model 940 that maps in vitro characteristics to in vivo characteristics. In one example, in vitro and in vivo characteristics may be correlated based on a linear regression or multiple linear regressions of the in vitro characteristics and the in vivo characteristics.

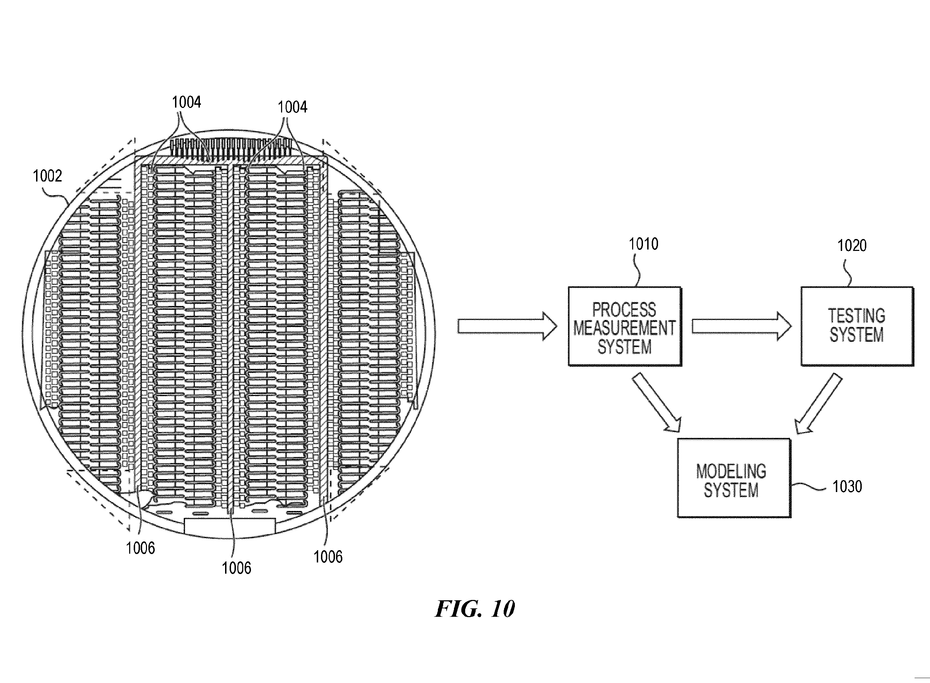

**FIG. 10** illustrates an example of determining in vitro and/or in vivo characteristics of glucose sensors using fabrication process measurement data according to certain embodiments. As illustrated, an array of electrochemical sensors may be fabricated on a substrate 1002, which may subsequently be singulated into individual sensors, for example, by dicing. As described above with respect to, for example, FIGS. 4A-4C, each electrochemical sensor may include multiple electrodes, and each electrode may include multiple layers. In the illustrated example, the array of electrochemical sensors may be concurrently fabricated in regions 1004 on substrate 1002, and additional process control monitors (PCM) may be concurrently fabricated in regions 1006 on substrate 1002. The PCMs may be adjacent to or otherwise in the vicinity of regions 1004. In the illustrated example, regions 1004 may be arranged in vertically-oriented columns on substrate 1002 with regions 1006 being arranged in vertically-oriented columns between regions 1004. Each region 1006 may include multiple PCMs (or sacrificial sensors) arranged vertically throughout the length of region 1006, while each region 1004 may include multiple electrochemical sensors arranged vertically throughout the length of region 1004.

During and/or after the fabrication process, PCMs fabricated within regions 1006 may be analyzed using a process measurement system 1010 (which may include one or more instruments or systems) to obtain fabrication process measurement data. Process measurement system 1010 may be capable of measuring the biological, chemical, electrical, and/or physical characteristics of the respective PCMs. The fabrication process measurement data obtained for each PCM or electrochemical sensor may include, but not limited to, glucose oxidase (GOx) thickness, GOx solution activity, glucose limiting membrane (GLM) thickness, working electrode (WE) platinum imaginary impedance, counter electrode (CE) platinum imaginary impedance, human serum albumin (HSA) concentration, and the like. It is noted that the fabrication process measurements are not limited to any particular type or number of fabrication process measurements listed, and may include measurements of any number of different properties or characteristics (e.g., dielectric characteristics, permeability, diffusivity, etc.). Additionally, or alternatively, in some embodiments, the fabrication process measurements may be obtained by directly measuring characteristics of the electrochemical sensors in regions 1004.

In some embodiments, some fabrication process measurements may be performed during the fabrication of the electrochemical sensors. For example, an imaginary impedance of the working electrode (and similarly, the imaginary impedance for the counter electrode) may be measured after a platinum electroplating process is performed to form electroplated layer 484. The GOx solution activity measurements may be performed during or after the GOx solution preparation process prior to deposition, while the GOx thickness (e.g., the thickness of Gox layer 488) may be measured after the slot coating and selective patterning processes over the working electrode 404. The HSA concentration may be measured during or after the solution preparation process prior to spray coating the HSA layer 490, and the GLM thickness (e.g., the thickness of GLM layer 494) may be measured after applying GLM layer 494. In some embodiments, these measurements may be performed with respect to the electrodes of sacrificial electrochemical sensors or PCMs fabricated in regions 1006 on substrate 1002. In some embodiments, additional fabrication process measurements such as surface roughness or other topographic measurements may be obtained for the electrodes during or after fabrication (e.g., via interferometry).

The fabrication process measurements obtained by process measurement system 1010 may be provided to a modeling system 1030. In some embodiments, modeling system 1030 may interpolate and/or extrapolate the fabrication process measurement data for different PCMs to obtain representative fabrication process measurement data for a given electrochemical sensor fabricated on substrate 1002. For example, modeling system 1030 may maintain an association between the location (e.g., the coordinates) of a respective PCM on substrate 1002 and the corresponding fabrication process measurement data obtained for the respective PCM. Thereafter, based on the location of a respective electrochemical sensor fabricated on substrate 1002, modeling system 1030 may identify a subset of PCMs that are neighboring, adjacent, or otherwise proximate to that respective electrochemical sensor, obtain the fabrication process measurement data for the identified subset of PCMs, and then average, interpolate, or otherwise combine the fabrication process measurement data for the different PCMs of the subset, based on a relationship between the location of the respective electrochemical sensor and the locations of the different PCMs to obtain representative fabrication process measurement data for the location of substrate 1002 where the respective electrochemical sensor is fabricated.

In some embodiments, each electrochemical sensor of a set of the electrochemical sensors fabricated in regions 1004 may be analyzed using a testing system 1020 (which may include one or more instruments or subsystems) to obtain in vitro and/or in vivo measurement results in response to one or more analyte samples with known glucose concentrations. For example, in some embodiments, electrochemical sensors fabricated in regions 1004 may be exposed to analyte samples with known concentrations of glucose, and testing system 1020 may include sensor electronics such as a potentiostat (e.g., as described above with respect to FIG. 5), a transmitter, a recorder, a current meter, a voltage meter, or other suitable measuring instruments capable of measuring the signals generated by or otherwise related to the electrochemical sensors. The in vitro measurement results obtained for each electrochemical sensor may include one or more of the electrical current output by the electrochemical sensor in response to test samples with known glucose concentrations, electrochemical impedance spectroscopy (EIS) values (for one or more frequencies) or other measurements indicative of a characteristic impedance associated with the electrochemical sensors in response to the test samples, counter voltage (Vcntr) (e.g., the difference between the counter electrode potential and working electrode potential), and the like. For example, the sensor electronics may include potentiostat hardware and firmware configured to cooperatively collect electrical current measurements corresponding to the electrical current flowing through the working electrode and resultant from reactions of the glucose oxidase layer(s) of the working electrode to the test samples, and determine the counter voltage (Vcntr) as the difference between the measured counter electrode potential and working electrode potential. The sensor electronics may also be configured to perform electrochemical impedance spectroscopy at various time intervals and using driving signals of multiple different frequencies. In some embodiments, a first electrochemical sensor may be used for in vitro measurements, while another electrochemical sensor adj acent to the first electrochemical sensor on substrate 1002 (and thus may have similar in vitro/in vivo performance as the first electrochemical sensor) may be used for in vivo measurements.

The in vitro and/or in vivo measurement results obtained by testing system 1020 may also be provided to modeling system 1030, which may maintain the in vitro and/or in vivo measurement results for respective electrochemical sensors fabricated on substrate 1002. Modeling system 1030 may also maintain an association between the in vitro and /or in vivo measurement results and representative fabrication process measurement data for each respective electrochemical sensor fabricated on substrate 1002. Based on the relationships between the fabrication process measurement data and the in vitro and/or in vivo measurement results for various electrochemical sensors, modeling system 1030 may determine models for predicting in vitro and/or in vivo characteristics (e.g., sensitivity and intercept or bias) for a electrochemical sensor as a function of one or more fabrication process measurement parameters associated with the electrochemical sensor.

For example, in some embodiments, for each characteristic of the vitro and/or in vivo characteristics of the electrochemical sensor, such as the sensitivity or intercept associated with Isig, modeling system 1030 may utilize machine learning or other techniques to determine the combination of fabrication process measurement parameters that may best correlate to or be predictive of the characteristic, and also determine a corresponding equation, function, or another model and corresponding weights or coefficients for predicting the feature based on the combination of fabrication process measurement parameters. The determined model may be capable of mapping a particular combination of one or more fabrication process measurement parameters to an in vitro or in vivo characteristic of the electrochemical sensor, such as the sensitivity or intercept associated with Isig, or another in vitro or in vivo characteristic associated with another signal of the electrochemical sensor, such as the EIS values, Vcntr, and the like.

In one example, modeling system 1030 may determine an in vitro sensitivity (e.g., in terms of the output sensor current Isig) of the respective electrochemical sensor based on the known glucose concentrations of test samples used for testing the respective electrochemical sensor and the measured in vitro electrical current outputs of the respective electrochemical sensor in response to the test samples. Thereafter, modeling system 1030 may utilize machine learning techniques to identify a subset of fabrication process measurement parameters that may be correlated to or predictive of the in vitro sensitivity of the electrochemical sensor to glucose concentration, and then determine a corresponding equation, function, or another model (e.g., a neural network) for predicting the in vitro sensitivity of the electrochemical sensor based on the subset of fabrication process measurement parameters. For example, the fabrication process measurement parameters for each electrochemical sensor may be structured into corresponding matrices or vectors and fed to the neural network as inputs to the neural network, and the output of the neural network may be compared with the determined in vitro sensitivity to determine a loss (or cost) function. A back-propagation technique may be used to modify the weight and/or bias associated with each fabrication process measurement parameter based on the loss function, thereby training the model to learn the appropriate input parameters and corresponding weights and/or bias for minimizing the loss function. Fabrication process measurement parameters that have little or no correlation with the in vitro sensitivity (e.g., associated with a very low weight in the neural network) may be pruned to simplify and/or optimize the model. Other characteristics of the vitro and/or in vivo characteristics of the electrochemical sensor may be modeled using the fabrication process measurement parameters in similar manners.

It is noted that any number of different machine learning techniques or other optimization techniques may be utilized to determine the models for predicting the in vitro and/or in vivo characteristics of the electrochemical sensor based on fabrication process measurement data, including, for example, genetic programming, support vector machines, Bayesian networks, probabilistic machine learning models, principal component analysis, fuzzy logic, heuristically derived combinations, and the like, or a combination thereof. In addition, electrochemical sensors on multiple different substrates may be used for the measurements and modeling to achieve the desired level of accuracy, reliability, and generality of the resultant models. Furthermore, as described above, the in vitro and/or in vivo characteristics, such as the sensitivity and intercept, may vary over time. Therefore, the in vitro and/or in vivo measurements for an electrochemical sensor may be performed at various time instants during the life time of the electrochemical sensor. As such, the models for predicting in vitro and/or in vivo characteristics (e.g., sensitivity and intercept or bias) for a electrochemical sensor as a function of one or more fabrication process measurement parameters associated with the electrochemical sensor may also use time (e.g., age or time in operation of the electrochemical sensor) as an input parameter.

In some embodiments, before the electrochemical sensor is shipped to the user (e.g., during or after the fabrication and/or assembly at the factory) or inserted into the user's body, in vitro characteristics of each electrochemical sensor as a function of the age of the electrochemical sensor may be predicted using the models and fabrication process measurement parameters, the predicted in vitro characteristics may then be mapped to in vivo characteristics based on the in vitro to in vivo translation models described above, and the in vivo characteristics of each electrochemical sensor may be saved to a storage device of the sensor electronics for the electrochemical sensor so that the in vivo characteristics may be used by the sensor electronics to determine SG values based on sensor measurement data (e.g., Isig, Vcntr, EIS, etc.). When the in vivo portion of the electrochemical sensor is inserted into the body of the user and the sensor electronics are activated, the in vivo characteristics of the electrochemical sensor may be retrieved from the storage device and used by the sensor electronics to estimate SG values based on sensor measurement data (e.g., Isig, Vcntr, EIS, etc.).

In some embodiments, fabrication process measurement parameters and the models for mapping the fabrication process measurement parameters to in vitro and/or in vivo characteristics (and models for mapping in vitro characteristics to in vivo characteristics) may be saved to a storage device of the sensor electronics for the electrochemical sensor, where the sensor electronics may determine the in vivo characteristics using the models and the fabrication process measurement parameters. In some embodiments, the in vitro characteristics of each electrochemical sensor as a function of the age of the electrochemical sensor may be predicted using fabrication process measurement parameters and the models determined in the learning phase, and saved with the in vitro to in vivo translation models to a storage device of the sensor electronics for the electrochemical sensor before the electrochemical sensor is shipped to the user or inserted into the user's body, and the sensor electronics may determine the in vivo characteristics using the in vitro to in vivo translation models and the in vitro characteristics. In some embodiments, a server may receive the fabrication process measurement data and predicted the in vivo characteristics of an electrochemical sensor directly from the fabrication process measurement data or in directly from the in vitro characteristics predicted using the fabrication process measurement data, and the predicted in vivo characteristics may be sent to the sensor electronics for the electrochemical sensor after the sensor electronics are activated. In some embodiments, the sensor electronics may send sensor measurement data (e.g., Isig, Vcntr, EIS, etc.) to a receiver device, such as a smart phone, an insulin pump, or a server, which may determine SG values based on in vivo characteristics (or the models and fabrication process measurement parameters) of the electrochemical sensor stored thereon or in a database.

Based on the predicted in vivo characteristics, one or more SG models may be used selectively or in combination to estimate SG values from sensor measurement data, where the one or more SG models may utilize the in vivo characteristics as part of the inputs. It is noted that the SG models that utilize estimated in vivo characteristics of the sensors for SG estimation are not limited to the functions or equations described above with respect to the examples. In some embodiments, the SG models may include other linear or nonlinear functions or equations, or may include various machine learning models (e.g., various neural network models). In addition, the SG models may use features derived from the sensor measurement data as described above, such as derivatives (e.g., rate of change) or integration of the sensor measurement data, historic sensor measurement data, filtered sensor measurement data, products of parameters of the sensor measurement data, and the like. In some embodiments, the SG models may use one or both of the in vivo sensitivity and in vivo intercept as input. In some embodiments, the SG models may use other in vivo characteristics that may be affected by the variation in fabrication processes and may be estimated using fabrication process measurement data.

As described above, since the in vivo sensitivity, in vivo intercept, and other in vivo characteristics may be estimated based on fabrication process measurement data associated with the specific sensor and models that map the fabrication process measurement data to in vivo characteristics directly or indirectly, using the estimated in vivo characteristics of the sensor in the SG models for determining SG values based on in vivo sensor measurement data (e.g., Isig, Vcntr, and EIS) may at least partially account for the effect of manufacturing process variability on the sensor measurement data. Therefore, the determined SG values may be more accurate and reliable. In addition, since the in vivo characteristics may be time-variant, sensor degradation over time may be at least partially accounted for by using the time-variant in vivo characteristics of the sensor in the SG models. Furthermore, no in vitro or in vivo calibration may be needed, which may reduce the burden on users.

In many cases, a single SG model may not be able to accurately estimate the blood glucose level because various sensor properties may affect the glucose level measurement results in complex ways that may be difficult to model using a single SG model. According to certain embodiments, a set of SG models may be used to estimate SG values from SG measurement data generated by a sensor, where the input parameter space of the set of SG models may be partitioned into a plurality of regions, and each SG model of the set of SG models may be a regional model used for input parameters that are within a respective region of the plurality of regions of the input parameter space. For example, in some embodiments, the input parameter space may be partitioned based on the age of the sensor, the Vcntr, the predicted in vivo sensitivity, the predicted in vivo intercept, or any combination thereof. In one example, the input parameter space may be divided based on two parameters, where at least one of the two parameters may include an in vivo characteristic, such as the in vivo sensitivity or in vivo intercept described above. In some other examples, the input parameter space may be divided based on three or more parameters, where at least one of the three or more parameters may include an in vivo characteristic, such as the in vivo sensitivity or in vivo intercept. The input parameter space may be partitioned uniformly or nonuniformly. The SG model for each region may be separately trained using different sets of training data, or may be trained together using a same set of training data. In some embodiments, the SG models may use the same input parameters and have similar structures, but may have different coefficients or weights for a same input parameter. In some embodiments, the SG models for different regions of the input parameter space may use different input parameters and/or may include different types of model or different model structures.

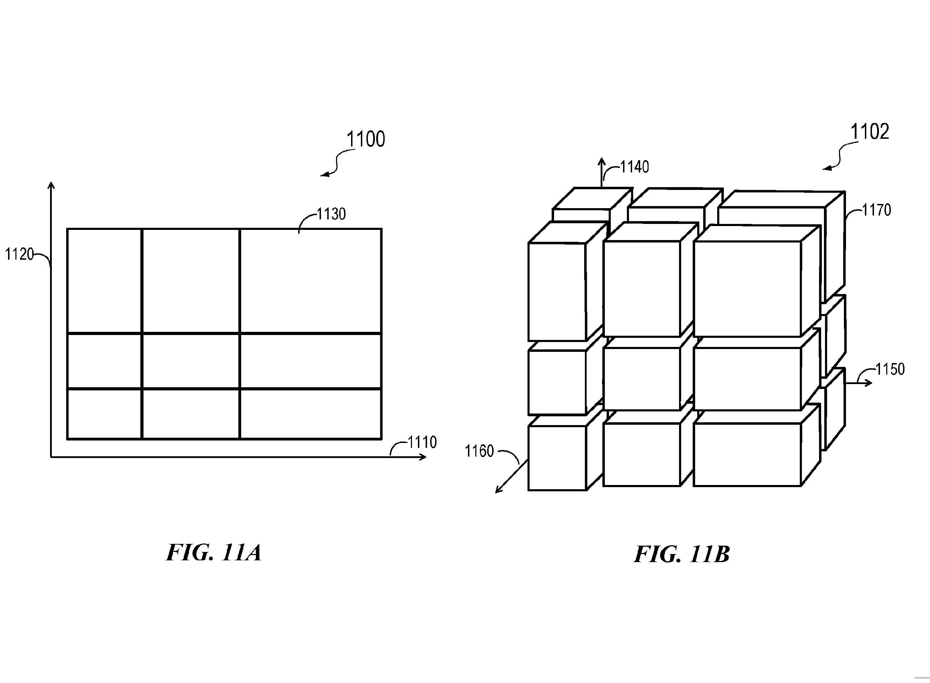

**FIG. 11A** shows an example of a partitioned model 1100 including a set of regional models for modeling a relationship between SG values and sensor measurement data according to certain embodiments. In the illustrated example, the input parameter space may be partitioned based on ranges of two parameters 1110 and 1120, where at least one of the two parameters may include in vivo sensitivity, in vivo intercept, or another predicted in vivo characteristic of a glucose sensor, and the other one of the two parameters may include, for example, sensor age, Vcntr, another in vivo characteristic of glucose sensors, another input parameter of the SG model, or an environmental property (e.g., moisture or temperature) of the glucose sensor. Each region 1130 of the input parameter space may be associated with a unique regional SG model. Regions 1130 may have the same or different sizes. Each region 1130 may have a regular shape (e.g., rectangle) or an irregular shape, where a boundary between two adjacent regions may be a straight line or may be a curve.

**FIG. 11B** shows another example of a partitioned model 1102 including a set of regional models for modeling a relationship between SG values and sensor measurement data according to certain embodiments. In the illustrated example, the input parameter space may be partitioned based on ranges of three or more parameters 1140, 1150, and 1160, where at least one of the three or more parameters may include in vivo sensitivity, in vivo intercept, or another predicted in vivo characteristic of the glucose sensor, and the other parameters of the three or more parameters may include, for example, the sensor age, Vcntr, another in vivo characteristic of glucose sensors, another input parameter of the SG model, or an environmental property (e.g., moisture or temperature) of the glucose sensor, or a combination thereof. Each region 1170 of the input parameter space may be associated with a unique regional SG model. Regions 1170 may have the same or different sizes. Each region 1170 may have a regular shape or irregular shape.

As shown in FIGS. 11A and 11B, the input parameter space may be partitioned into a plurality of regions that collectively forms the contiguous input parameter space. In some embodiments, to partition the input parameter space, each region of the input parameter space in which the sensor may have similar characteristics or may be modeled using a similar model may be identified. In some embodiments, the stability of sensor trends, regularity of sensor signal magnitudes, or other characteristics of the sensor signal may be identified and used to partition the input parameter space. In some embodiments, machine learning techniques, such as clustering, dimensionality reduction (e.g., principal component analysis (PCA)), and the like, may be used to partition the input parameter space. In some embodiments, the partition and/or the models may be modified if the models used in one or more regions generate erroneous SG values (e.g., having a large Mean Absolute Relative Difference (MARD)), if the models for adjacent regions output significantly different SG values for similar input parameters, and the like.

In some embodiments, different models may be used in different regions 1130 or 1170 of the input parameter space. The models may include, for example, a linear regression model, a neural network, another machine learning model, a function, an equation, or another model. In some embodiments, a plurality of candidate models for each region of the input parameter space may be ranked based on the complexity, accuracy, and/or other metrics of the models, and one or more models may be selected for the region based on the ranking. This process may be performed for each region. In some embodiments, simpler models may be sufficient for certain regions while more complex models may be needed for other regions. Therefore, a combination of different types of models may be used.

In some embodiments, various smoothing or blending techniques may be used in the regional models of adjacent regions, to avoid abrupt changes in output SG values at boundaries between adjacent regions caused by the different models used for the adjacent regions. For example, in some embodiments, the output SG value for input parameters near a boundary between two regions may be a weighted average of the output SG values from the two models for the two adjacent regions. The output SG value for input parameters at an area (e.g., a corner) adjacent to three or more adjacent regions may be a weighted average of the output SG values from the three or more models for the three or more adjacent regions. In other embodiments, other techniques for smoothing or blending the output SG values for input parameters at or near the region boundaries may be used.

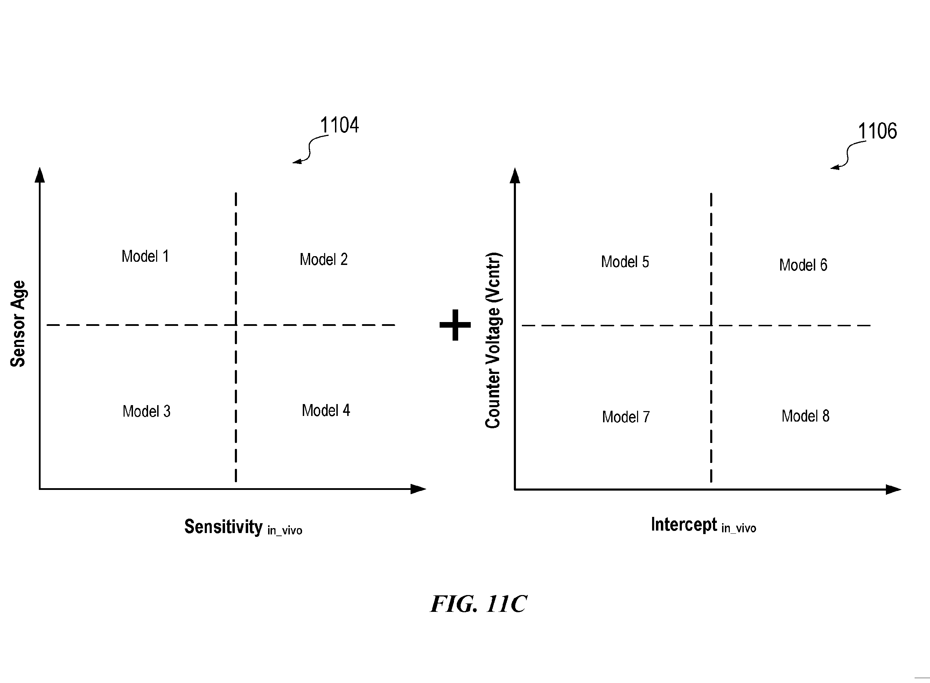

FIG. 11C illustrates another example of an ensembled model including multiple sets of regional SG models for estimating SG values based on sensor measurement data generated by a glucose sensor according to certain embodiments. As shown in FIG. 11C, the input parameter space may be divided differently according to different partitioning schemes (e.g., based on different sets of input parameters, and/or dividing the ranges of one or more input parameters differently), where at least one of the input parameters used in at least one partitioning scheme may include predicted in-vivo sensitivity, in vivo intercept, or another predicted in vivo characteristic of glucose sensors. A respective set of regional SG models may be determined for regions of the input parameter space divided according to each partitioning scheme. One regional SG model may be selected from each set of regional SG models for a respective input parameter space partitioning scheme, for example, based on the region in which the current input parameters fall. Therefore, multiple regional SG models may be selected from the multiple sets of regional SG models for the different partitioning schemes and may be used to calculate the SG values in parallel, where the outputs of the selected multiple regional SG models may be combined (e.g., by averaging or weighted averaging) to determine a final predicted SG value.

In one example shown in FIG. 11C, in a first partitioning scheme 1104, the input parameter space may be divided into four regions based on different ranges of sensor age and predicted in vivo sensitivity, and four different regional SG models (e.g., models 1-4) may be determined (e.g., trained) for the four regions, respectively. In a second partitioning scheme 1106, the input parameter space may be divided into four regions based on different ranges of Vcntr and predicted in vivo intercept, and four different regional SG models (e.g., models 5-8) may be determined (e.g., trained) for the four regions, respectively. When a set of input parameters (e.g., sensor measurement data and derivative features) is received, a first regional SG model may be selected from the four regional SG models for the four regions in first partitioning scheme 1104 based on the region that the current sensor age and predicted in vivo sensitivity both fall in, and a second regional SG model may be selected from the four regional SG models for the four regions in second partitioning scheme 1106 based on the region that the current Vcntr and predicted in vivo intercept both fall in. The SG value predicted using the first regional SG model and the SG value predicted using the second regional SG model may be combined (e.g., averaged or weighted averaged) to determine a final SG value for the current input parameter values. It is noted that FIG. 11C is for illustrative purposes only, and is not intended to limit the partition schemes to the specific examples shown in FIG. 11C.

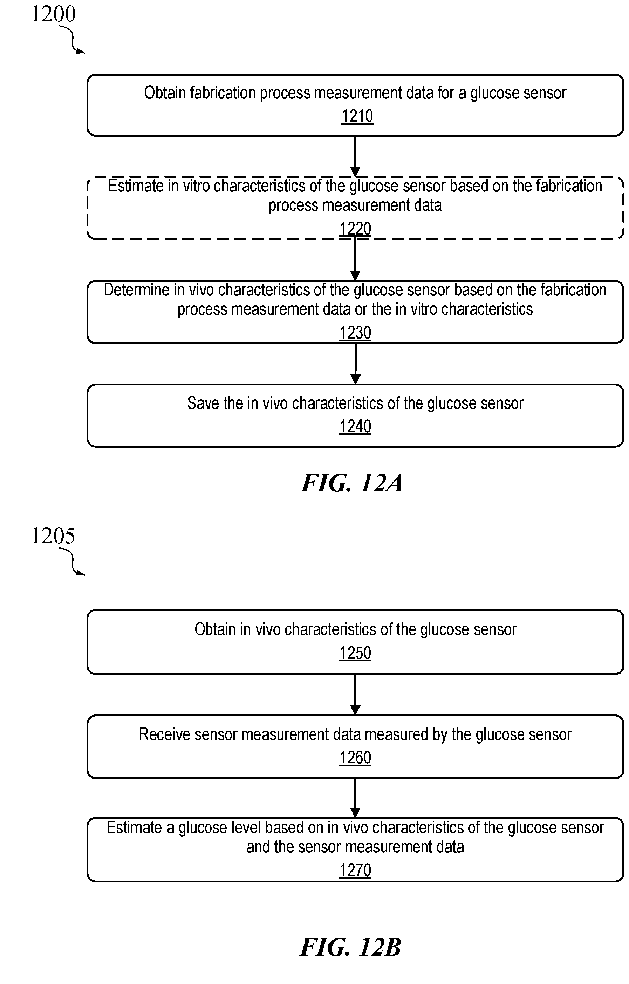

FIG. 12A includes a flowchart 1200 illustrating an example of a process of predicting in vivo characteristics of a glucose sensor according to certain embodiments. Operations in flowchart 1200 may be performed by, for example, a computing device (e.g., a computer or a server). In one example, operations in flowchart 1200 may be performed by modeling system 1030 described above. Although flowchart 1200 may describe the operations as a sequential process, some of the operations may be performed in parallel or concurrently. In addition, the order of the operations may be rearranged. The process may have additional steps not included in the figure. Some operations may be optional or may be omitted.

Operations in block 1210 of flowchart 1200 may include obtaining fabrication process measurement data for the glucose sensor, as described above with respect to, for example, FIG. 10. The fabrication process measurement data for the glucose sensor may be measured during or after the fabrication of the glucose sensor, and may be measured at the location of the glucose sensor or one or more PCMs adjacent to the glucose sensor on a substrate. In some embodiments, the fabrication process measurement data may be calculated (e.g., interpolated or extrapolated) based on fabrication process measurement data for adjacent PCMs or sacrificial sensors. The fabrication process measurement data may include, for example, glucose oxidase (GOx) thickness, GOx solution activity, glucose limiting membrane (GLM) thickness, working electrode (WE) platinum imaginary impedance, counter electrode (CE) platinum imaginary impedance, human serum albumin (HSA) concentration, dielectric characteristics, permeability, diffusivity, and the like.

Optional operations in block 1220 may include estimating in vitro characteristics of the glucose sensor based on the fabrication process measurement data. As described above with respect to, for example, FIG. 10, modeling system 1030 may utilize machine learning or other techniques to determine the combination of fabrication process measurement parameters that may best correlate with or be predictive of each characteristic of the in vitro characteristics, and also determine a corresponding equation, function, or another model and corresponding weights or coefficients for predicting the in vitro characteristic based on the combination of fabrication process measurement parameters. The determined models may be capable of mapping a respective combination of one or more fabrication process measurement parameters to each in vitro characteristic of the glucose sensor, such as the in vitro sensitivity or intercept associated with Isig. The determined models may be used to predict the in vitro characteristics of new glucose sensors based on the fabrication process measurement data associated with the new glucose sensors.

Operations in block 1230 may include determining in vivo characteristics of the glucose sensor based on the fabrication process measurement data or the in vitro characteristics estimated in block 1220. For example, in some embodiments, the in vivo characteristics of the glucose sensor may be directly predicted based on the fabrication process measurement data using a determined model that maps a particular combination of one or more fabrication process measurement parameters to an in vivo characteristic of the glucose sensor, such as the in vivo sensitivity or intercept associated with Isig, as described above with respect to FIG. 10. In some embodiments, the in vivo characteristics of the glucose sensor may be predicted using an in vitro to in vivo translation model as described above and the in vitro characteristics estimated in block 1220.

Operations in block 1240 may include saving the in vivo characteristics of the glucose sensor, for example, into a memory device of the sensor electronics connected to the glucose sensor, before the glucose sensor is sent to a user. For example, the in vivo characteristics of the glucose sensor may be predicted using operations of block 1210-1230, and saved into the memory device after the glucose sensor is assembled with the sensor electronics in the factory. The in vivo characteristics of the glucose sensor saved in the memory device may be read and used by a processing unit after the glucose sensor is inserted into the user and initialized and the sensor electronics are activated.

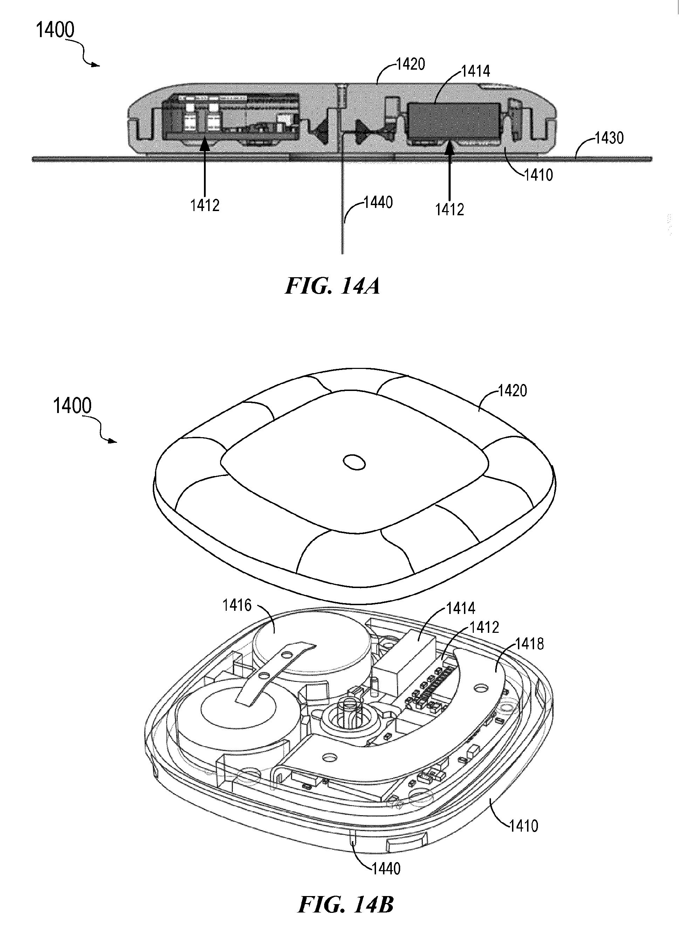

**FIG. 12B** includes a flowchart 1205 illustrating an example of a process of using in vivo characteristics of a glucose sensor and one or more SG models to estimate SG values according to certain embodiments. Operations in flowchart 1205 may be performed by, for example, monitoring device 104, glucose sensor subsystem 210, sensor electronics device 304, a glucose sensor device 1400 described below with respect to FIGS. 14A-14B, and the like. Although flowchart 1205 may describe the operations as a sequential process, some of the operations may be performed in parallel or concurrently. In addition, the order of the operations may be rearranged. The process may have additional steps not included in the figure. Some operations may be optional or may be omitted.

Operations in block 1250 of flowchart 1205 may include obtaining in vivo characteristics of a glucose sensor predicted using fabrication process measurement data associated with the glucose sensor as described above with respect to, for example, FIGS. 10 and 12A. The in vivo characteristics may include, for example, an in vivo sensitivity, an in vivo intercept, another in vivo characteristic of the glucose sensor, or a combination thereof. The in vivo sensitivity and the in vivo intercept may characterize a relationship between a sensor current (Isig) of the glucose sensor and a corresponding blood glucose (BG) level according to:

$$BG = Isig \,/\, \text{in vivo sensitivity} + \text{in vivo intercept}$$

Both the in vivo sensitivity and the in vivo intercept of the glucose sensor may be time-variant. In some embodiments, the above equation may include an optional function of one or more other (predicted) in vivo features. As described above, the in vivo characteristics of the glucose sensor may be stored in a memory device of the sensor electronics of the glucose sensor prior to insertion of the glucose sensor into a subcutaneous layer of a user, and the sensor electronics may obtain the in vivo characteristics of the glucose sensor by reading the in vivo characteristics from the memory device of the glucose sensor.

Operations in block 1260 may include receiving sensor measurement data measured by the glucose sensor. The sensor measurement data may include, for example, sensor current (Isig), counter voltage (Vcntr), electrochemical impedance spectroscopy (EIS) data, an age of the glucose sensor, or a combination thereof. In some embodiments, derivative features or other features may be derived or extracted from the sensor measurement data as described above with respect to, for example, FIGS. 6-8, and may be used, in combination with the sensor measurement data, as input parameters for estimating SG values.

Operations in block 1270 may include estimating an SG value using an SG model, where input parameters of the SG model may include the in vivo characteristics of the glucose sensor and the sensor measurement data (and features derived or extracted from the sensor measurement data), and an output of the SG model may be the SG value. In some embodiments, the input parameters of the SG model may include one or both of the predicted in vivo sensitivity and the predicted in vivo intercept of the glucose sensor. In some embodiments, the SG model may include a partitioned model comprising a plurality of regional models for respective regions of a plurality of regions of an input parameter space of the SG model, where estimating the SG value using the SG model may include selecting one regional model from the plurality of regional models based on the in vivo characteristics of the glucose sensor, the sensor measurement data, or both. Different regions of the plurality of regions of the input parameter space of the SG model may correspond to different ranges of the input parameters of the SG model. In some embodiments, the input parameter space of the SG model may be partitioned into the plurality of regions based at least in part on the in vivo characteristics of the glucose sensor. The SG model may include one or more machine learning models, equations, functions, or a combination thereof.

In some embodiments, the SG model may include a first partitioned model comprising a first plurality of regional models for respective regions of a first plurality of regions of an input parameter space of the SG model, where the input parameter space of the SG model may be partitioned into the first plurality of regions based on a first partition scheme. The SG model may also include a second partitioned model comprising a second plurality of regional models for respective regions of a second plurality of regions of the input parameter space of the SG model, where the input parameter space of the SG model may be partitioned into the second plurality of regions based on a second partition scheme that is different from the first partition scheme. For example, the first partition scheme and the second partition scheme may partition the input parameter space using different combinations of input parameters, and/or may partition the input parameter space according to different combinations of input parameter ranges. The SG value may be estimated using the SG model by: generating a first SG value using one regional model selected from the first plurality of regional models based on the in vivo characteristics of the glucose sensor, the sensor measurement data, or both; generating a second SG value using one regional model selected from the second plurality of regional models based on the in vivo characteristics of the glucose sensor, the sensor measurement data, or both; and determining a final SG value based on a combination (e.g., an average or weighted average) of the first SG value and the second SG value.

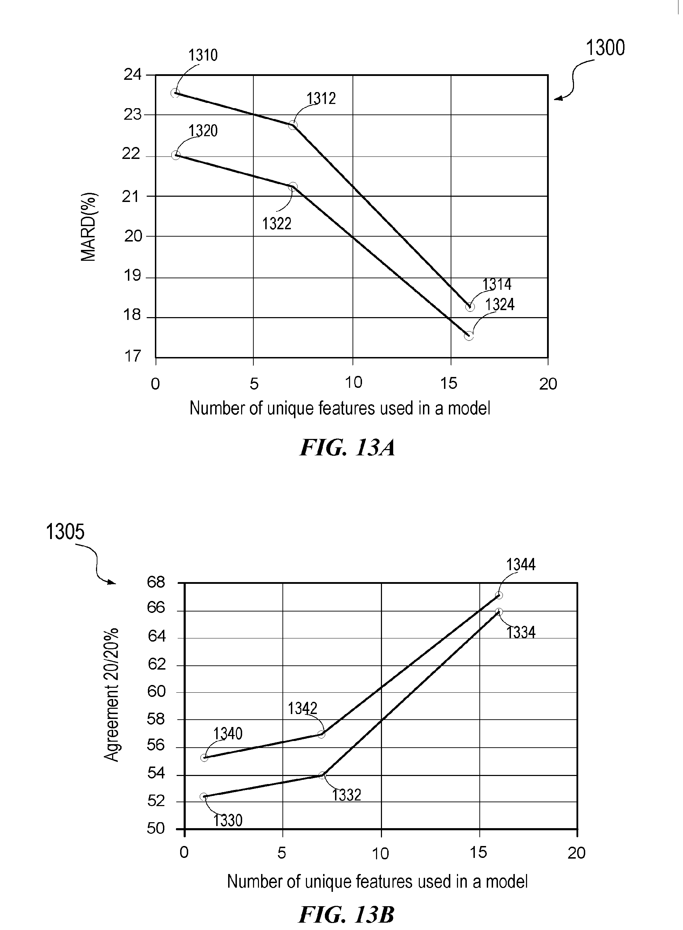

FIG. 13A includes a diagram 1300 illustrating examples of improving sensor performance by using predicted in vivo characteristics in SG value estimation according to certain embodiments. In FIG. 13A, the horizontal axis represents the number of unique features used in an SG model, and the vertical axis represents the Mean Absolute Relative Difference (MARD, which indicates the accuracy of the sensors) of the estimated SG values with respect to reference measurement results. In the illustrated examples, data points 1310, 1312, and 1314 indicate MARD values of SG values estimated using SG models that use 1, 7, and 16 unique parameters of sensor measurement data, respectively, without using predicted in vivo characteristics. Data points 1320, 1322, and 1324 indicate MARD values of SG values estimated using SG models that use the same 1, 7, and 16 unique parameters of the same sensor measurement data as data points 1310, 1312, and 1314, and also use predicted in vivo characteristics (e.g., sensitivity and intercept associated with Isig). As illustrated, the MARD values and thus the accuracy of the sensor may be significantly improved by using the predicted in vivo characteristics as additional input parameters of the SG models.

FIG. 13B includes a diagram 1305 illustrating examples of improving sensor performance by using predicted in vivo characteristics in SG value estimation according to certain embodiments. In FIG. 13B, the horizontal axis represents the number of unique features used in an SG model, and the vertical axis represents the proportion of estimated SG values that are within 20 mg/dL from reference glucose levels or within 20% from the reference glucose levels (referred to as agreement 20/20%, which may indicate the accuracy of glucose sensors). In the illustrated examples, data points 1330, 1332, and 1334 indicate agreement 20/20% values of SG values estimated using SG models that use 1, 7, and 16 unique parameters of sensor measurement data, respectively, without using predicted in vivo characteristics. Data points 1340, 1342, and 1344 indicate agreement 20/20% values of SG values estimated using SG models that use the same 1, 7, and 16 unique parameters of the same sensor measurement data as data points 1330, 1332, and 1334, and also use predicted in vivo characteristics (including sensitivity and intercept). As illustrated, the agreement 20/20% values and thus the accuracy of the sensor may be significantly improved by using the predicted in vivo characteristics as additional input parameters of the SG models.

FIGS. 14A and 14B depict an example of an integrated glucose sensor device 1400 according to certain embodiments, which may implement some of the examples disclosed herein. Glucose sensor device 1400 may be an example of monitoring device 104, glucose sensor subsystem 210, or CGM system 300. In the illustrated example, glucose sensor device 1400 may include a housing formed by a base 1410 and a top cover 1420, sensor electronics enclosed in the housing, an electrochemical sensor 1440 partially within the housing and connected to the sensor electronics, and an adhesive patch 1430 attached to base 1410. Electrochemical sensor 1440 may be an example of the electrochemical sensors described above (e.g., glucose sensor 400 or 402), and may include multiple electrodes at a distal end that may be inserted into the subcutaneous layer of a user. A proximal end of electrochemical sensor 1440 may be bent and held against a printed circuit board (PCB) 1412 by a elastomer or rubber block 1414, such that contacts on the proximal end of electrochemical sensor 1440 may be physically and electrically connected to contact pads on PCB 1412. The housing may be hermetically sealed to protect the sensor electronics from moisture. Electrochemical sensor 1440 may be inserted into the subcutaneous layer of the user using an inserter. The inserter may include a needle and may enclose the entire glucose sensor device 1400 before insertion. When activated (e.g., pushed), the inserter may move the entire glucose sensor device 1400 toward the skin of the user to attach adhesive patch 1430 to the skin of the user and insert the distal end of electrochemical sensor 1440 into the subcutaneous layer of the user using the needle, where the needle may be retracted, for example, automatically after the distal end of electrochemical sensor 1440 is inserted into the user.

The sensor electronics in the housing may include batteries 1416, an antenna 1418, and circuits on PCB 1412. The circuits on PCB 1412 may include, for example, a processor, a controller, a potentiostat, filters, amplifiers, a wireless (e.g., Bluetooth) transceiver, one or more memory devices, power conversion and management devices, or a combination thereof. Predicted in vivo characteristics of electrochemical sensor 1440 and SG model(s) described above may be stored in the one or more memory devices. Before or after the distal end of electrochemical sensor 1440 is inserted into the subcutaneous layer of the user, the sensor electronics may be activated to initialize the sensor for measuring sensor measurement data (e.g., Isig, Vcntr, EIS, etc.). The processor or controller may execute instruction code stored on the one or more memory device to obtain the sensor measurement data, process (e.g., filter, amplify, transform, etc.) the sensor measurement data, estimate SG values using the SG models and sensor measurement data, optionally blank certain estimated SG values, and transmit, via the wireless transceiver and antenna 1418, the estimated SG values to a receiver (e.g., a smartphone or an insulin pump), as described above.

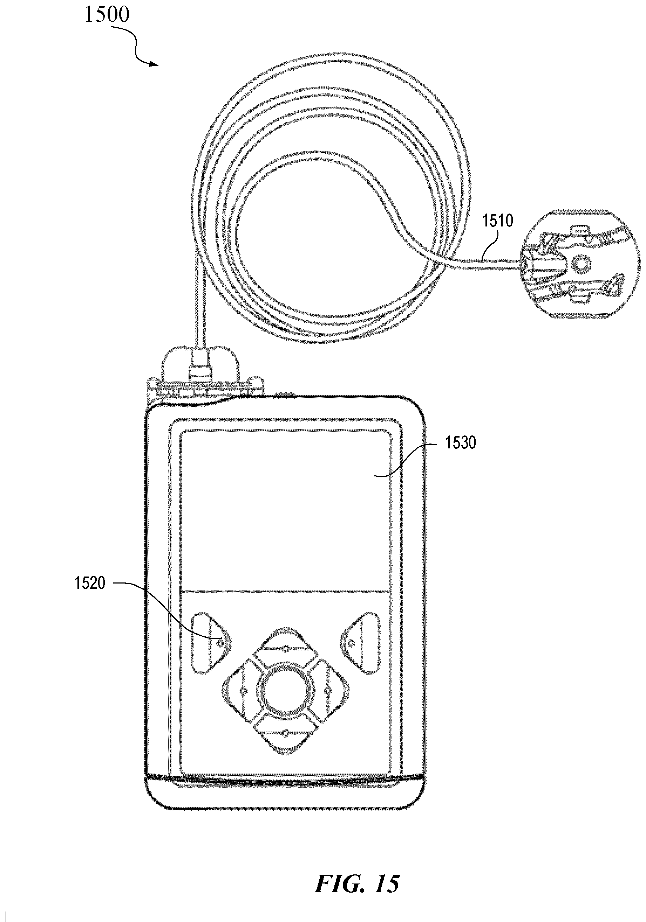

**FIG. 15** depicts an example of a delivery device 1500, which may implement a delivery device described above (e.g., delivery device 102 or insulin delivery subsystem 230). In an insulin delivery device implemented using delivery device 1500, insulin delivery may be performed based on internal communication between a central computing module (e.g., a microprocessor of delivery device 1500) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., computing device 106 of FIG. 1) communicatively coupled to a remote or cloud computing system (e.g., remote/cloud computing system 108 of FIG. 1). Delivery device 1500 may communicate various event data toward the remote or cloud computing system, which may communicate insulin delivery determinations toward delivery device 1500, in accordance with the techniques described herein.

Delivery device 1500 may provide insulin through a small tube 1510 configured for fluidic connection with a cannula inserted subcutaneously. Delivery device 1500 may be configured to deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. In the depicted example, delivery device 1500 includes a user interface having button elements 1520 that can be manipulated to administer a bolus of insulin, to change therapy settings, to change user preferences, to select display features, and the like. Delivery device 1500 may also include a display device 1530 that can be used to present various types of information or data to the user. In accordance with aspects of the present disclosure, a user of delivery device 1500 may use button elements 1520 to input certain event data (e.g., event type, event start time, event details, etc.), and the user inputs can be confirmed using display device 1530. Delivery device 1500 of FIG. 15 is merely provided by way of example, and other types of insulin delivery devices and other techniques different from those described above are contemplated to be within the scope of the present disclosure.

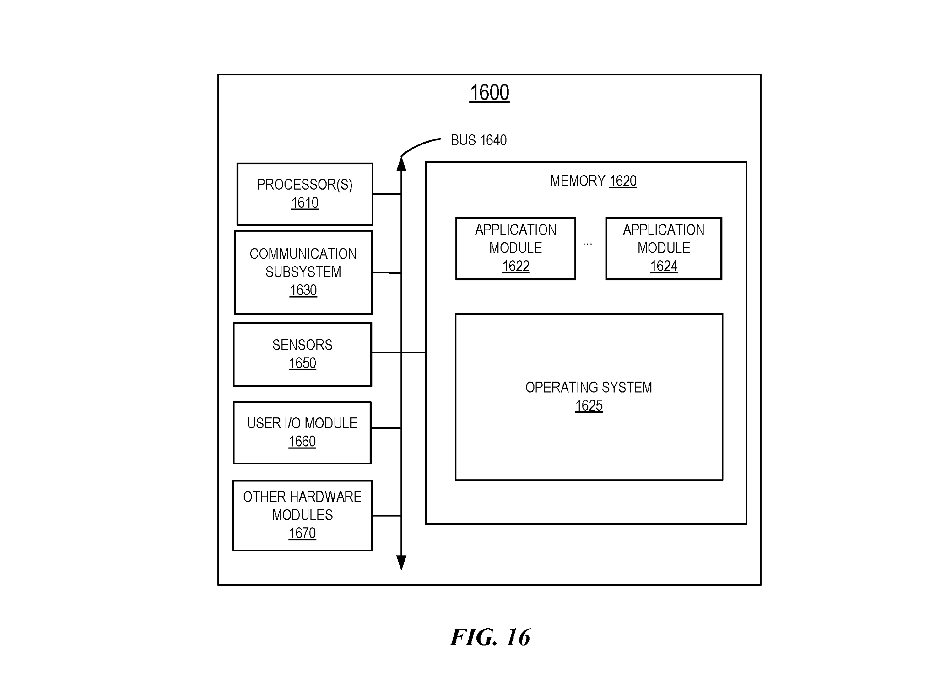

**FIG. 16** is a block diagram of an example of an electronic device 1600 that may implement some of the examples disclosed herein. For example, electronic device 1600 may be used to implement delivery device 102, monitoring device 104, computing device 106, and remote/cloud computing system 108, glucose sensor subsystem 210, controller 220, insulin delivery subsystem 230, glucose delivery subsystem 240, and glucagon delivery subsystem 250, CGM system 300, glucose sensor device 1400, delivery device 1500, or another device that may be used to calculate insulin dose and/or predict future blood glucose levels. In the illustrated example, electronic device 1600 may include one or more processor(s) 1610 and memory 1620. Processor(s) 1610 may be configured to execute instructions stored in memory 1620 for performing one or more of the methods described herein and other applications. Processor(s) 1610 may include, for example, one or more central processing units, microprocessors, microcontrollers, special-purpose processors (e.g., digital signal processors), ASICs, DSPs, FPGAs, or other processors suitable for implementation within a portable electronic device. Processor(s) 1610 may be communicatively coupled with a plurality of components within electronic device 1600. To realize this communicative coupling, processor(s) 1610 may communicate with the other illustrated components across a bus 1640. Bus 1640 may be any subsystem adapted to transfer data within electronic device 1600. Bus 1640 may include a plurality of computer buses and additional circuitry to transfer data.

Memory 1620 may include one or more transitory and/or non-transitory storage devices, such as, for example, a static random-access memory (SRAM), a dynamic random access memory (DRAM), a read-access memory (RAM), a read-only memory (ROM), a programmable read-only memory (PROM), an erasable programmable read-only memory (EPROM), a FLASH-EPROM, a secure digital (SD) card, and any other memory chip or cartridge. Such storage devices may be configured to implement any appropriate data stores, including without limitation, various file systems, data structures, computer-readable instructions, program modules, and the like. In some embodiments, memory 1620 may be distributed into different hardware modules.

Memory 1620 may include an operating system 1625 loaded therein. Operating system 1625 may be operable to initiate the execution of the instructions provided by application modules 1622-1824 and/or manage other hardware modules 1670, as well as interface with a communication subsystem 1630 which may include one or more wired and/or wireless transceivers. Operating system 1625 may be adapted to perform other operations across the components of electronic device 1600 including threading, virtualization, resource management, data storage control, and other similar functionality. In some embodiments, memory 1620 may store a plurality of application modules 1622 through 1624, which may include any number of applications. Examples of the applications may include an insulin calculator, a blood glucose level monitor or predictor, a glucose level management application, and the like. Application modules 1622-1424 may include particular instructions to be executed by processor(s) 1610. In some embodiments, certain applications or parts of application modules 1622-1424 may be executable by other hardware modules 1670.

Communication subsystem 1630 may include, for example, an infrared communication device, a wireless communication device and/or chipset (such as a Bluetooth® device, an IEEE 802.11 device, a Wi-Fi device, a WiMax device, cellular communication devices, etc.), and/or similar communication interfaces. One or more antennas (not shown) may be used for wireless communication as part of communication subsystem 1630 or as a separate component coupled to any portion of electronic device 1600, such as a wireless charging receiver or a near-field communication receiver. In some embodiments, communication subsystem 1630 may include circuits for wired communication technologies, such as Ethernet, coaxial communications, universal serial bus (USB), and the like. Communications subsystem 1630 may permit data to be exchanged with a network, other computer systems, and/or any other devices. For example, communications subsystem 1630 may be used to receive therapy determinations for therapeutic fluid (e.g., insulin) delivery, such as from a cloud computing system via an intermediary computing device (e.g., a controller) communicatively coupled to electronic device 1600, where processor(s) 1610 may, based on the therapy determinations, send commands to an actuator controller to cause the delivery of appropriate amounts of therapeutic fluid (e.g., insulin) to a user. In another example, communications subsystem 1630 may be used to communicate measurement results (e.g., sensor glucose levels) to a computing device (e.g., a smartphone or a personal health monitoring device) and/or to a remote server via the computing device, or receive data (e.g., calibration data, configuration data, etc.) from the computing device or the remote server via the computing device.

Sensor(s) 1650 may include, for example, a camera, an infrared sensor, an accelerometer, a pressure sensor, a temperature sensor, a proximity sensor, a magnetometer, a gyroscope, an inertial sensor (e.g., an inertial measurement unit (IMU)), an ambient light sensor, a position sensor, a depth sensor, a gesture detector, or any other similar module operable to provide sensory output and/or receive sensory input. In one example, sensor(s) 1650 may be used to implement a meal detector.

Input/output user interface 1660 may allow a user to send action requests to electronic device 1600 to perform particular actions, and may provide information (e.g., status of electronic device 1600, measurement results, alerts, etc.) to the user. Input/output user interface 1660 may include one or more input devices, such as, for example, a touchscreen, a touch pad, microphone(s), button(s), dial(s), switch(es), a keyboard, a mouse, a game controller, or any other suitable device for receiving action requests and communicating the received action requests to processor(s) 1610. In some embodiments, input/output user interface 1660 may include one or more output devices, such as a display, a speaker, a light emitting device, a haptic device, and the like, to provide feedback or alarm to the user.

In some embodiments, electronic device 1600 may include a plurality of other hardware modules 1670. Each of other hardware modules 1670 may be a physical module within electronic device 1600. While each of other hardware modules 1670 may be permanently configured as a structure, some of other hardware modules 1670 may be temporarily configured to perform specific functions or temporarily activated. Examples of other hardware modules 1670 may include, for example, an audio output and/or input module (e.g., a microphone or speaker), a near field communication (NFC) module, a rechargeable battery, a battery management system, a wired/wireless battery charging system, an actuator controller, and the like. In some embodiments, one or more functions of other hardware modules 1670 may be implemented in software.

In one example, electronic device 1600 may be part of an insulin delivery device (e.g., a pump) that can deliver fast-acting insulin through a small tube configured for fluidic connection with a cannula inserted subcutaneously. Electronic device 1600 may cause the delivery of two types of dosages-a basal dosage, which can be delivered periodically (e.g., every five minutes) in tiny amounts throughout the day and night, and a bolus dosage to cover an increase in blood glucose from meals and/or to correct high blood glucose levels. The insulin delivery device may include a user interface having button elements that can be manipulated to administer a bolus of insulin, to change therapy settings, to change user preferences, to select display features, and the like. The insulin delivery device may also include a display device that can be used to present various types of information or data to the user. In accordance with aspects of the present disclosure, a user of the insulin delivery device may use the button elements to input certain event data (e.g., event type, event start time, event details, etc.), and the user inputs can be confirmed using the display device.

In various implementations, the above-described hardware and modules may be implemented on a single device or on multiple devices that can communicate with one another using wired or wireless connections. In alternative configurations, different and/or additional components may be included in electronic device 1600. Similarly, functionality of one or more of the components can be distributed among the components in a manner different from the manner described above.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the spirit and scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure. While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. The aspects and features of the present disclosure and may be embodied in various forms. Thus, specific structural and functional details disclosed herein are not to be interpreted as limiting, but as a basis for the claims and as a representative basis for teaching one skilled in the art to variously employ the present disclosure in any appropriately detailed structure.

With reference to the appended figures, components that can include memory can include non-transitory machine-readable media. The term "machine-readable medium," "processor-readable medium," or "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of' if used to associate a list, such as A, B, or C, can be interpreted to mean A, B, C, or any combination of A, B, and/or C, such as AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

In view of this description embodiments may include different combinations of features. Implementation examples are described in the following numbered clauses:

**Clause 1.** A processor-implemented method comprising:

obtaining in vivo characteristics of a glucose sensor predicted using fabrication process measurement data associated with the glucose sensor, the in vivo characteristics including an in vivo sensitivity, an in vivo intercept, or a combination thereof;

receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

**Clause 2.** The processor-implemented method of Clause 1, wherein the in vivo sensitivity and the in vivo intercept characterize a relationship between a sensor current (Isig) of the glucose sensor and a corresponding blood glucose level (BG) according to:

BG = Isig / in vivo sensitivity + in vivo intercept + an optional function of one or more other in vivo features.

**Clause 3.** The processor-implemented method of Clause 1 or 2, wherein the input parameters of the SG model includes both the in vivo sensitivity and the in vivo intercept of the glucose sensor.

**Clause 4.** The processor-implemented method of any of Clauses 1-3, wherein both the in vivo sensitivity and the in vivo intercept of the glucose sensor are time-variant.

**Clause 5.** The processor-implemented method of any of Clauses 1-4, wherein:

the in vivo characteristics of the glucose sensor are stored in a memory device of the glucose sensor prior to insertion of the glucose sensor into a subcutaneous layer of a user; and

obtaining the in vivo characteristics of the glucose sensor comprises reading the in vivo characteristics of the glucose sensor from the memory device of the glucose sensor.

**Clause 6.** The processor-implemented method of any of Clauses 1-5, further comprising generating derived features based on the sensor measurement data, wherein the input parameters of the SG model include the derived features.

**Clause 7.** The processor-implemented method of any of Clauses 1-6, wherein:

the SG model includes a partitioned model comprising a plurality of regional models for respective regions of a plurality of regions of an input parameter space of the SG model; and

estimating the SG value using the SG model includes selecting one regional model from the plurality of regional models based on the in vivo characteristics of the glucose sensor, the sensor measurement data, or both.

**Clause 8.** The processor-implemented method of Clause 7, wherein different regions of the plurality of regions of the input parameter space of the SG model correspond to different ranges of the input parameters of the SG model.

**Clause 9.** The processor-implemented method of Clause 8, wherein the input parameter space of the SG model is partitioned into the plurality of regions based at least in part on the in vivo characteristics of the glucose sensor.

**Clause 10.** The processor-implemented method of any of Clauses 1-6, wherein:

the SG model includes a first partitioned model comprising a first plurality of regional models for respective regions of a first plurality of regions of an input parameter space of the SG model, wherein the input parameter space of the SG model is partitioned into the first plurality of regions based on a first partition scheme;

the SG model further includes a second partitioned model comprising a second plurality of regional models for respective regions of a second plurality of regions of the input parameter space of the SG model, wherein the input parameter space of the SG model is partitioned into the second plurality of regions based on a second partition scheme that is different from the first partition scheme; and

estimating the SG value using the SG model includes:

generating a first SG value using one regional model selected from the first plurality of regional models based on the in vivo characteristics of the glucose sensor, the sensor measurement data, or both;

generating a second SG value using one regional model selected from the second plurality of regional models based on the in vivo characteristics of the glucose sensor, the sensor measurement data, or both; and

determining a final SG value based on a combination of the first SG value and the second SG value.

**Clause 11.** The processor-implemented method of Clause 10, wherein at least one of the first partition scheme or the second partition scheme partitions the input parameter space based at least in part on the in vivo characteristics of the glucose sensor.

**Clause 12.** The processor-implemented method of any of Clauses 1-11, wherein:

the in vivo characteristics of the glucose sensor are determined based on in vitro characteristics of the glucose sensor and an in vitro to in vivo translation model; and
the in vitro characteristics of the glucose sensor are predicted based on the fabrication process measurement data associated with the glucose sensor.

**Clause 13.** The processor-implemented method of any of Clauses 1-12, wherein the SG model includes one or more machine learning models, equations, functions, or a combination thereof.

**Clause 14.** A system comprising:

one or more processors; and
one or more processor-readable media storing instructions which, when executed by the one or more processors, cause performance of operations including:

obtaining in vivo characteristics of a glucose sensor predicted using fabrication process measurement data associated with the glucose sensor, the in vivo characteristics including an in vivo sensitivity, an in vivo intercept, or a combination thereof;
receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and
estimating a sensor glucose (SG) value using an SG model, wherein input parameters of the SG model include the in vivo characteristics of the glucose sensor and the sensor measurement data, and the SG value is an output of the SG model.

**Clause 15.** The system of Clause 14, wherein the in vivo sensitivity and the in vivo intercept characterize a relationship between a sensor current (Isig) of the glucose sensor and a corresponding blood glucose level (BG) according to:

BG = Isig / in vivo sensitivity + in vivo intercept + an optional function of one or more other in vivo features.

**Clause 16.** The system of Clause 14 or 15, wherein both the in vivo sensitivity and the in vivo intercept of the glucose sensor are time-variant.

**Clause 17.** The system of any of Clauses 14-16, wherein:

the in vivo characteristics of the glucose sensor are stored in a memory device of the glucose sensor prior to insertion of the glucose sensor into a subcutaneous layer of a user; and
obtaining the in vivo characteristics of the glucose sensor comprises reading the in vivo characteristics of the glucose sensor from the memory device of the glucose sensor.

**Clause 18.** The system of any of Clauses 14-17, wherein the operations further comprise generating derived features based the sensor measurement data, wherein the input parameters of the SG model include the derived features.

**Clause 19.** The system of any of Clauses 14-18, wherein:

the SG model includes a partitioned model comprising a plurality of regional models for respective regions of a plurality of regions of an input parameter space of the SG model; and
estimating the SG value using the SG model includes selecting one regional model from the plurality of regional models based on the in vivo characteristics of the glucose sensor, the sensor measurement data, or both..

**Clause 20.** One or more non-transitory processor-readable media storing instructions which, when executed by one or more processors, cause performance of operations comprising:

obtaining in vivo characteristics of a glucose sensor predicted using fabrication process measurement data associated with the glucose sensor, the in vivo characteristics including an in vivo sensitivity, an in vivo intercept, or a combination thereof;
receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

Further disclosed herein is the subject-matter of the following items:

1. A processor-implemented method comprising:

obtaining in vivo characteristics of a glucose sensor predicted using fabrication process measurement data associated with the glucose sensor, the in vivo characteristics including an in vivo sensitivity, an in vivo intercept, or a combination thereof;

receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

2. The processor-implemented method of item 1, wherein the in vivo sensitivity and the in vivo intercept characterize a relationship between a sensor current (Isig) of the glucose sensor and a corresponding blood glucose level (BG) according to:

BG = Isig / in vivo sensitivity + in vivo intercept + an optional function of one or more other in vivo features.

3. The processor-implemented method of item 1 or 2, wherein the input parameters of the SG model include both the in vivo sensitivity and the in vivo intercept of the glucose sensor.

4. The processor-implemented method of any of items 1-3, wherein both the in vivo sensitivity and the in vivo intercept of the glucose sensor are time-variant.

5. The processor-implemented method of any of items 1-4, wherein:

the in vivo characteristics of the glucose sensor are stored in a memory device of the glucose sensor prior to insertion of the glucose sensor into a subcutaneous layer of a user; and

obtaining the in vivo characteristics of the glucose sensor comprises reading the in vivo characteristics of the glucose sensor from the memory device of the glucose sensor.

6. The processor-implemented method of any of items 1-5, further comprising generating derived features based on the sensor measurement data, wherein the input parameters of the SG model include the derived features.

7. The processor-implemented method of any of items 1-6, wherein:

the SG model includes a partitioned model comprising a plurality of regional models for respective regions of a plurality of regions of an input parameter space of the SG model; and

estimating the SG value using the SG model includes selecting one regional model from the plurality of regional models based on the in vivo characteristics of the glucose sensor, the sensor measurement data, or both.

8. The processor-implemented method of item 7, wherein different regions of the plurality of regions of the input parameter space of the SG model correspond to different ranges of the input parameters of the SG model.

9. The processor-implemented method of item 7 or 8, wherein the input parameter space of the SG model is partitioned into the plurality of regions based at least in part on the in vivo characteristics of the glucose sensor.

10. The processor-implemented method of any of items 1-6 or of any of items 1-9, wherein:

the SG model includes a first partitioned model comprising a first plurality of regional models for respective regions of a first plurality of regions of an input parameter space of the SG model, wherein the input parameter space of the SG model is partitioned into the first plurality of regions based on a first partition scheme;

the SG model further includes a second partitioned model comprising a second plurality of regional models for respective regions of a second plurality of regions of the input parameter space of the SG model, wherein the input parameter space of the SG model is partitioned into the second plurality of regions based on a second partition scheme that is different from the first partition scheme; and

estimating the SG value using the SG model includes:

generating a first SG value using one regional model selected from the first plurality of regional models based on the in vivo characteristics of the glucose sensor, the sensor measurement data, or both;

generating a second SG value using one regional model selected from the second plurality of regional models based on the in vivo characteristics of the glucose sensor, the sensor measurement data, or both; and

determining a final SG value based on a combination of the first SG value and the second SG value.

11. The processor-implemented method of item 10, wherein at least one of the first partition scheme or the second partition scheme partitions the input parameter space based at least in part on the in vivo characteristics of the glucose sensor.

12. The processor-implemented method of any of items 1-11, wherein:

the in vivo characteristics of the glucose sensor are determined based on in vitro characteristics of the glucose sensor and an in vitro to in vivo translation model; and

the in vitro characteristics of the glucose sensor are predicted based on the fabrication process measurement data associated with the glucose sensor.

13. The processor-implemented method of any of items 1-12, wherein the SG model includes one or more machine learning models, equations, functions, or a combination thereof.

14. One or more non-transitory processor-readable media storing instructions which, when executed by one or more processors, cause performance of operations comprising:

obtaining in vivo characteristics of a glucose sensor predicted using fabrication process measurement data associated with the glucose sensor, the in vivo characteristics including an in vivo sensitivity, an in vivo intercept, or a combination thereof;

receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

15. A system comprising:

one or more processors; and

one or more processor-readable media storing instructions which, when executed by the one or more processors, cause performance of operations including:

obtaining in vivo characteristics of a glucose sensor predicted using fabrication process measurement data associated with the glucose sensor, the in vivo characteristics including an in vivo sensitivity, an in vivo intercept, or a combination thereof;

receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

**Claims**

1. A processor-implemented method comprising:

   obtaining in vivo characteristics of a glucose sensor predicted using fabrication process measurement data associated with the glucose sensor, the in vivo characteristics including an in vivo sensitivity, an in vivo intercept, or a combination thereof;

   receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

   estimating a sensor glucose (SG) value using an SG model, wherein input parameters of the SG model include the in vivo characteristics of the glucose sensor and the sensor measurement data, and the SG value is an output of the SG model.

2. The processor-implemented method of claim 1, wherein the in vivo sensitivity and the in vivo intercept characterize a relationship between a sensor current (Isig) of the glucose sensor and a corresponding blood glucose level (BG) according to:

   BG = Isig / in vivo sensitivity + in vivo intercept + an optional function of one or more other in vivo features.

3. The processor-implemented method of claim 1 or 2, wherein the input parameters of the SG model include both the in vivo sensitivity and the in vivo intercept of the glucose sensor.

4. The processor-implemented method of any of claims 1-3, wherein both the in vivo sensitivity and the in vivo intercept of the glucose sensor are time-variant.

5. The processor-implemented method of any of claims 1-4, wherein:

   the in vivo characteristics of the glucose sensor are stored in a memory device of the glucose sensor prior to insertion of the glucose sensor into a subcutaneous layer of a user; and

   obtaining the in vivo characteristics of the glucose sensor comprises reading the in vivo characteristics of the glucose sensor from the memory device of the glucose sensor.

6. The processor-implemented method of any of claims 1-5, further comprising generating derived features based on the sensor measurement data, wherein the input parameters of the SG model include the derived features.

7. The processor-implemented method of any of claims 1-6, wherein:

   the SG model includes a partitioned model comprising a plurality of regional models for respective regions of a plurality of regions of an input parameter space of the SG model; and

   estimating the SG value using the SG model includes selecting one regional model from the plurality of regional models based on the in vivo characteristics of the glucose sensor, the sensor measurement data, or both.

8. The processor-implemented method of claim 7, wherein different regions of the plurality of regions of the input parameter space of the SG model correspond to different ranges of the input parameters of the SG model.

9. The processor-implemented method of claim 7 or 8, wherein the input parameter space of the SG model is partitioned into the plurality of regions based at least in part on the in vivo characteristics of the glucose sensor.

10. The processor-implemented method of any of claims 1-6, wherein:

   the SG model includes a first partitioned model comprising a first plurality of regional models for respective regions of a first plurality of regions of an input parameter space of the SG model, wherein the input parameter space of the SG model is partitioned into the first plurality of regions based on a first partition scheme;

   the SG model further includes a second partitioned model comprising a second plurality of regional models for respective regions of a second plurality of regions of the input parameter space of the SG model, wherein the input parameter space of the SG model is partitioned into the second plurality of regions based on a second partition scheme that is different from the first partition scheme; and

estimating the SG value using the SG model includes:

generating a first SG value using one regional model selected from the first plurality of regional models based on the in vivo characteristics of the glucose sensor, the sensor measurement data, or both;

generating a second SG value using one regional model selected from the second plurality of regional models based on the in vivo characteristics of the glucose sensor, the sensor measurement data, or both; and

determining a final SG value based on a combination of the first SG value and the second SG value.

11. The processor-implemented method of claim 10, wherein at least one of the first partition scheme or the second partition scheme partitions the input parameter space based at least in part on the in vivo characteristics of the glucose sensor.

12. The processor-implemented method of any of claims 1-11, wherein:

the in vivo characteristics of the glucose sensor are determined based on in vitro characteristics of the glucose sensor and an in vitro to in vivo translation model; and

the in vitro characteristics of the glucose sensor are predicted based on the fabrication process measurement data associated with the glucose sensor.

13. The processor-implemented method of any of claims 1-12, wherein the SG model includes one or more machine learning models, equations, functions, or a combination thereof.

14. One or more non-transitory processor-readable media storing instructions which, when executed by one or more processors, cause performance of operations comprising:

obtaining in vivo characteristics of a glucose sensor predicted using fabrication process measurement data associated with the glucose sensor, the in vivo characteristics including an in vivo sensitivity, an in vivo intercept, or a combination thereof;

receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

15. A system comprising:

one or more processors; and

one or more processor-readable media storing instructions which, when executed by the one or more processors, cause performance of operations including:

obtaining in vivo characteristics of a glucose sensor predicted using fabrication process measurement data associated with the glucose sensor, the in vivo characteristics including an in vivo sensitivity, an in vivo intercept, or a combination thereof;

receiving sensor measurement data measured by the glucose sensor, the sensor measurement data including sensor current (Isig), counter voltage (Vcntr), electrochemical impedance spectroscopy (EIS) data, an age of the glucose sensor, or a combination thereof; and

estimating a sensor glucose (SG) value using an SG model, wherein input parameters of the SG model include the in vivo characteristics of the glucose sensor and the sensor measurement data, and the SG value is an output of the SG model.

**FIG. 1**

**FIG. 2**

*FIG. 3*

**FIG. 4A**

**FIG. 4B**

**FIG. 4C**

FIG. 5

*FIG. 6*

*FIG. 7*

**FIG. 8**

**IN VIVO**

920 — OBTAIN IN VIVO MEASUREMENTS

922 — IDENTIFY IN VIVO FEATURES

**IN VITRO**

910 — OBTAIN IN VITRO MEASUREMENTS

912 — IDENTIFY IN VITRO FEATURES

930 — CORRELATE IN VITRO AND IN VIVO FEATURES

940 — IN VITRO TO IN VIVO TRANSLATION MODEL

*FIG. 9*

**FIG. 10**

EP 4 555 927 A1

FIG. 11B

FIG. 11A

*FIG. 11C*

1200

Obtain fabrication process measurement data for a glucose sensor
1210

Estimate in vitro characteristics of the glucose sensor based on the fabrication process measurement data
1220

Determine in vivo characteristics of the glucose sensor based on the fabrication process measurement data or the in vitro characteristics
1230

Save the in vivo characteristics of the glucose sensor
1240

*FIG. 12A*

1205

Obtain in vivo characteristics of the glucose sensor
1250

Receive sensor measurement data measured by the glucose sensor
1260

Estimate a glucose level based on in vivo characteristics of the glucose sensor and the sensor measurement data
1270

*FIG. 12B*

FIG. 13A

FIG. 13B

1400

1420  1414

1430

1412  1440  1412  1410

**FIG. 14A**

1400

1420

1416  1414
1412
1418

1410

1440

**FIG. 14B**

1500

1510

1530

1520

**FIG. 15**

**FIG. 16**

**EUROPEAN SEARCH REPORT**

Application Number

EP 24 20 8150

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/209497 A1 (WANG LIANG [US] ET AL) 8 July 2021 (2021-07-08) * figure 5 * * paragraphs [0103], [0107], [0116] - [0123], [0129], [0131], [0138] - [0141], [0145], [0151] * | 1-15 | INV. A61B5/145 A61B5/1495 |
| A | US 2022/233108 A1 (AJEMBA PETER [US] ET AL) 28 July 2022 (2022-07-28) * paragraphs [0051] - [0054] * | 7-11 | |

TECHNICAL FIELDS SEARCHED (IPC)

A61B

The present search report has been drawn up for all claims

| Place of search | Date of completion of the search | Examiner |
|---|---|---|
| The Hague | 20 March 2025 | Albrecht, Ronald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8150

This annex lists the patent family members relating to the patent documents cited in  the above-mentioned European search report.
The members are as contained in the European Patent Office EDP file on
The European Patent Office is in no way liable for these particulars which are merely given for the purpose of information.

20-03-2025

| Patent document cited in search report | Publication date | Patent family member(s) | | Publication date |
|---|---|---|---|---|
| US 2021209497 A1 | 08-07-2021 | AU | 2020418515 A1 | 25-08-2022 |
| | | EP | 4084675 A1 | 09-11-2022 |
| | | US | 2021209497 A1 | 08-07-2021 |
| | | US | 2024020558 A1 | 18-01-2024 |
| | | WO | 2021138168 A1 | 08-07-2021 |
| US 2022233108 A1 | 28-07-2022 | US | 2022233108 A1 | 28-07-2022 |
| | | US | 2022233109 A1 | 28-07-2022 |

EPO FORM P0459

For more details about this annex : see Official Journal of the European Patent Office, No. 12/82

**EP 4 555 927 A1**

**Patent documents cited in the description**

- US 773422